# EUROPEAN PATENT APPLICATION

(11) **EP 4 389 759 A1**
(43) Date of publication of application: **26.06.2024**
(21) Application number: 22858843.0
(22) Date of filing: 21.08.2022
(51) Int. Cl.: C07K 14/075, C07K 14/005, C12N 15/861, C12N 7/01, C12N 15/09, A61K 48/00

(54) **ISOLATED MODIFIED AAV5 CAPSID PROTEIN VP1**

(30) Priority: 20.08.2021 RU 2021124727
(71) Applicant: Joint Stock Company "Biocad", Saint Petersburg 198515 (RU)
(72) Inventor: STRELKOVA, Anna Nikolaevna, Kirovskaya obl., 612261 (RU); LEGOTSKII, Sergei Aleksandrovich, Moscow, 107370 (RU); SHUGAEVA, Tatiana Evgenievna, Moscow, 119021 (RU); GERSHOVICH, Pavel Mikhailovich, Saint Petersburg, 198328 (RU); PROKOFYEV, Alexander Vladimirovich, Saint Petersburg, 198206 (RU); PEREPELKINA, Mariya Pavlovna, Saint Petersburg, 198328 (RU); IAKOVLEV, Pavel Andreevich, Saint Petersburg, 190068 (RU); MOROZOV, Dmitry Valentinovich, Saint-Petersburg, 190000 (RU)
(74) Representative: Meissner Bolte Partnerschaft mbB
(86) International application number: PCT/RU2022/050257
(87) International publication number: WO 2023/022633

(57) **Abstract**

The present application relates to the fields of gene therapy and molecular biology. More specifically, the present invention relates to an isolated modified capsid protein VP1 from adeno-associated virus serotype 5 (AAV5), comprising one or more amino acid substitutions compared to the wild-type AAV5 capsid protein VP1, which substitutions increase the efficiency of transduction, increase the efficiency of packaging of AAV viral genomes with rAAV5-based products, and also increase the efficiency of production (assembly) of a vector based on recombinant adeno-associated virus serotype 5 (rAAV5), to a capsid and a vector based on the above VP1, as well as to uses thereof.

## Description

### FIELD OF THE INVENTION

The present application relates to the fields of gene therapy and molecular biology. More specifically, the present invention relates to an isolated modified capsid protein VP1 from adeno-associated virus serotype 5 (AAV5), comprising one or more amino acid substitutions compared to the wild-type AAV5 capsid protein VP1, which substitutions increase the efficiency of transduction, increase the efficiency of packaging of AAV viral genomes with rAAV5-based products, and also increase the efficiency of production (assembly) of a vector based on recombinant adeno-associated virus serotype 5 (rAAV5), to a capsid and a vector based on the above VP1, as well as to uses thereof.

### BACKGROUND OF THE INVENTION

Adeno-associated virus (AAV) is a small (25 nm), independent replication-defective, nonenveloped virus. Many different AAV serotypes have been described in human and primates. The adeno-associated virus genome is composed of (+ or -) single-stranded DNA (ssDNA) being about 4,700 nucleotides long. At the ends of a genomic DNA molecule there are accommodated terminal inverted repeats (ITRs). The genome comprises two open reading frames (ORFs): Rep and Cap comprising several alternative reading frames encoding various protein products. The rep products are essential for AAV replication, whereas three capsid proteins (VP1, VP2, and VP3), along with other alternative products, are encoded by the Cap gene. VP1, VP2, and VP3 proteins are present at 1:1:10 ratio to form an icosahedral capsid (Xie Q. et al. The atomic structure of adeno-associated virus (AAV-2), a vector for human gene therapy. Proc Natl Acad Sci USA, 2002; 99:10405-10410). During recombinant AAV (rAAV) vector production, an expression cassette flanked by ITR is packaged into an AAV capsid. The genes required for AAV replication are not included in the cassette. Recombinant AAV is considered to be one of the safest and most widely used viral vectors for *in vivo* gene transfer. Vectors can infect cells of multiple tissue types to provide strong and sustained transgene expression. They are also non-pathogenic, and have a low immunogenicity profile (High KA et al., "rAAV human trial experience" Methods Mol Biol. 2011; 807:429-57).

One of the urgent purposes of research in the area of development of effective gene therapy is optimization of vectors to improve certain properties of the given vectors.

The various AAV serotypes are characterized by affinity for distinct host cell surface receptors, toward which they have tropism. Thus, the primary known receptor for AAV2 is heparan sulfate proteoglycan, the coreceptors are integrin heterodimer aVβ5, fibroblast growth factor receptor type 1, and hepatocyte growth factor receptor, c-Met. AAV12 binds to heparan sulfate proteoglycans and sialic acid. AAV4 and AAV5 bind to N- and O-linked sialic acids, respectively. AAV5 activates the platelet-derived growth factor receptor. Further, a relationship has been established between the amino acid sequence of AAV capsid proteins and the process of assembly thereof, encapsidation of the genome, affinity for different types of receptors represented on the surface of host cells (Govindasamy L. et. al. Structural insights into adeno-associated virus serotype 5. J Virol. 2013 Oct;87(20):11187-99).

The international application WO2012145601 describes adeno-associated virus (AAV) virions with variant capsid protein, wherein the AAV virions exhibit greater infectivity of retinal cells, when administered via intravitreal injection, compared to wild-type AAV.

The international application WO2013158879 describes an adeno-associated virus (AAV) vector for delivering to a subject a heterologous nucleic acid sequence comprising the capsid protein VP1 comprising one or more lysine substitutions, wherein one lysine substitution is K137R, wherein said lysine substitution is effective for inhibiting ubiquitination of said capsid protein, thereby increasing transduction efficiency of said AVV vector in target cells.

To date, there is a need for AAV with improved properties compared to wild-type AAV, for example, which have increased transduction ability, increased efficiency of packaging of AAV viral genomes, as well as increased efficiency of generation due to highly efficient production (assembly) of encapsidated viral vectors based on recombinant adeno-associated virus (rAAV). Improved tissue transduction enables to minimize the dose of vector being administered to a subject.

### DESCRIPTION OF THE INVENTION

The authors of the invention have surprisingly found that the presence of one or more amino acid substitutions in the wild-type AAV5 capsid protein VP1, which are selected from the group:
G226V,
S2A, G226V and T711S,
T614A,
S2A, T614A and T711S,
T614V,
S2A, T614V and T711S,
caused an increase in the efficiency of transduction of target cells using the AAV serotype 5-based vector with this/these modification(s) and a significant increase in the efficiency of transgene delivery by the rAAV-based vectors with the above mutations.

The authors of the invention have surprisingly found that the presence of one or more amino acid substitutions in the wild-type AAV5 capsid protein VP1, which are selected from the group:
G226V,
S2A, G226V and T71 1S,
T614A,
S2A, T614A and T711S,
T614V,
S2A, T614V and T711S,
causes increased efficiency of packaging of AAV viral genomes with rAAV5-based products.

The authors of the invention have surprisingly found that the presence of one or more amino acid substitutions in the wild-type AAV5 capsid protein VP1, which are selected from the group:
G226V,
S2A, G226V and T71 1S,
T614A,
S2A, T614A and T711S,
T614V,
S2A, T614V and T711S,
causes increased production (assembly) of encapsidated viral vectors based on recombinant adeno-associated virus serotype 5 (rAAV5) as compared to the assembly of encapsidated viral vectors based on wild-type rAAV5 (free of the above mutations), i.e. the assembly of encapsidated viral vectors based on rAAV5 comprising the above modifications in the AAV5 capsid results in the production of viral vectors based on rAAV5 which contain a transgene (encapsidated heterologous nucleic acid) significantly more often as compared to the assembly of encapsidated viral vectors based on wild-type rAAV5 (free of the above mutations).

### Brief description of the invention

In one aspect, the present invention relates to an isolated modified capsid protein VP1 from adeno-associated virus serotype 5 (AAV5) for production of viral vectors based on recombinant adeno-associated virus serotype 5 (rAAV5), which comprises a wild-type AAV5 capsid protein VP1 amino acid sequence encoded by the Cap gene with one or more substitutions that are selected from the group:
G226V,
S2A, G226V and T71 1S,
T614A,
S2A, T614A and T711S,
T614V,
S2A, T614V and T711S,
wherein the wild-type AAV5 capsid protein VP1 amino acid sequence has the amino acid sequence represented by SEQ ID NO: 1.

In some embodiments of the invention, the isolated modified AAV5 capsid protein VP1 comprises a substitution at position G226V.

In some embodiments of the invention, the isolated modified AAV5 capsid protein VP1 has the amino acid sequence represented by SEQ ID NO: 3.

In some embodiments of the invention, the isolated modified AAV5 capsid protein VP1 comprises substitutions S2A, G226V and T711S.

In some embodiments of the invention, the isolated modified AAV5 capsid protein VP1 has the amino acid sequence represented by SEQ ID NO: 4.

In some embodiments of the invention, the isolated modified AAV5 capsid protein VP1 comprises the substitution T614A.

In some embodiments of the invention, the isolated modified AAV5 capsid protein VP1 has the amino acid sequence represented by SEQ ID NO: 5.

In some embodiments of the invention, the isolated modified AAV5 capsid protein VP1 comprises substitutions S2A, T614A and T711S.

In some embodiments of the invention, the isolated modified AAV5 capsid protein VP1 has the amino acid sequence represented by SEQ ID NO: 6.

In some embodiments of the invention, the isolated modified AAV5 capsid protein VP1 comprises the substitution T614V.

In some embodiments of the invention, the isolated modified AAV5 capsid protein VP1 has the amino acid sequence represented by SEQ ID NO: 7.

In some embodiments of the invention, the isolated modified AAV5 capsid protein VP1 comprises substitutions S2A, T614V and T711S.

In some embodiments of the invention, the isolated modified AAV5 capsid protein VP1 has the amino acid sequence represented by SEQ ID NO: 8.

In one aspect, the present invention relates to an isolated nucleic acid that encodes any of the above modified capsid proteins VP1 from adeno-associated virus serotype 5 that are used for production of viral vectors based on recombinant adeno-associated virus serotype 5.

In some embodiments of the invention, the isolated nucleic acid encodes a modified capsid protein VP1 from adeno-associated virus serotype 5 with the amino acid substitution G226V, which is represented by a nucleic sequence with SEQ ID NO: 11 or by any other sequence encoding the respective amino acid sequence.

In some embodiments of the invention, the isolated nucleic acid encodes a modified capsid protein VP1 from adeno-associated virus serotype 5 with the amino acid substitutions S2A, G226V and T711S and is represented by a nucleic sequence with SEQ ID NO: 12 or by any other sequence encoding the respective amino acid sequence.

In some embodiments of the invention, the isolated nucleic acid encodes a modified capsid protein VP1 from adeno-associated virus serotype 5 with the amino acid substitution T614A and is represented by a nucleic sequence with SEQ ID NO: 13 or by any other sequence encoding the respective amino acid sequence.

In some embodiments of the invention, the isolated nucleic acid encodes a modified capsid protein VP1 from adeno-associated virus serotype 5 with the amino acid substitutions S2A, T614A and T711S and is represented by a nucleic sequence with SEQ ID NO: 14 or by any other sequence encoding the respective amino acid sequence.

In some embodiments of the invention, the isolated nucleic acid encodes a modified capsid protein VP1 from adeno-associated virus serotype 5 with the amino acid substitution T614V and is represented by a nucleic sequence with SEQ ID NO: 15 or by any other sequence encoding the respective amino acid sequence.

In some embodiments of the invention, the isolated nucleic acid encodes a modified capsid protein VP1 from adeno-associated virus serotype 5 with the amino acid substitutions S2A, T614V and T711S and is represented by a nucleic sequence with SEQ ID NO: 16 or by any other sequence encoding the respective amino acid sequence.

In one aspect, the present invention relates to an isolated capsid for production of viral vectors based on recombinant adeno-associated virus serotype 5, comprising any of the above modified capsid proteins VP1 from adeno-associated virus serotype 5.

In some embodiments of the invention, the isolated capsid includes any of the above modified capsid proteins VP1 from adeno-associated virus serotype 5, AAV5 capsid protein VP2 or a modified variant thereof, and AAV5 capsid protein VP3 or a modified variant thereof.

In some embodiments of the invention, the isolated capsid includes a wild-type AAV5 capsid protein VP2.

In some embodiments of the invention, the isolated capsid includes a wild-type AAV5 capsid protein VP2, which has the amino acid sequence represented by SEQ ID NO: 17.

In some embodiments of the invention, the isolated capsid includes a modified capsid protein VP2 from adeno-associated virus serotype 5.

In some embodiments of the invention, the isolated capsid includes a modified AAV5 capsid protein VP2, which comprises the substitution G90V.

In some embodiments of the invention, the isolated capsid includes a modified AAV5 capsid protein VP2, which comprises the substitution G90V, and has the amino acid sequence represented by SEQ ID NO: 19.

In some embodiments of the invention, the isolated capsid includes a modified AAV5 capsid protein VP2, which comprises the substitutions G90V and T575S.

In some embodiments of the invention, the isolated capsid includes a modified AAV5 capsid protein VP2, which comprises the substitutions G90V and T575S, and has the amino acid sequence represented by SEQ ID NO: 20.

In some embodiments of the invention, the isolated capsid includes a modified AAV5 capsid protein VP2, which comprises the substitution T478A.

In some embodiments of the invention, the isolated capsid includes a modified AAV5 capsid protein VP2, which comprises the substitution T478A, and has the amino acid sequence represented by SEQ ID NO: 21.

In some embodiments of the invention, the isolated capsid includes a modified AAV5 capsid protein VP2, which comprises the substitutions T478A and T575S.

In some embodiments of the invention, the isolated capsid includes a modified AAV5 protein, which comprises the substitutions T478A and T575S, and has the amino acid sequence represented by SEQ ID NO: 22.

In some embodiments of the invention, the isolated capsid includes a modified AAV5 capsid protein VP2, which comprises the substitution T478V.

In some embodiments of the invention, the isolated capsid includes a modified AAV5 capsid protein VP2, which comprises the substitution T478V, and has the amino acid sequence represented by SEQ ID NO: 23.

In some embodiments of the invention, the isolated capsid includes a modified AAV5 capsid protein VP2, which comprises the substitutions T478V and T575S.

In some embodiments of the invention, the isolated capsid includes a modified AAV5 capsid protein VP2, which comprises the substitutions T478V and T575S, and has the amino acid sequence represented by SEQ ID NO: 24.

In some embodiments of the invention, the isolated capsid includes a wild-type AAV5 capsid protein VP3.

In some embodiments of the invention, the isolated capsid includes a wild-type AAV5 capsid protein VP3, which has the amino acid sequence represented by SEQ ID NO: 33.

In some embodiments of the invention, the isolated capsid includes a modified capsid protein VP3 from adeno-associated virus serotype 5.

In some embodiments of the invention, the isolated capsid includes a modified AAV5 capsid protein VP3, which comprises the substitution G34V.

In some embodiments of the invention, the isolated capsid includes a modified AAV5 capsid protein VP3, which comprises the substitution G34V, and has the amino acid sequence represented by SEQ ID NO: 35.

In some embodiments of the invention, the isolated capsid includes a modified AAV5 capsid protein VP3, which comprises the substitutions G34V and T519S.

In some embodiments of the invention, the isolated capsid includes a modified AAV5 capsid protein VP3, which comprises the substitutions G34V and T519S, and has the amino acid sequence represented by SEQ ID NO: 36.

In some embodiments of the invention, the isolated capsid includes a modified AAV5 capsid protein VP3, which comprises the substitution T422A.

In some embodiments of the invention, the isolated capsid includes a modified AAV5 capsid protein VP3, which comprises the substitution T422A, and has the amino acid sequence represented by SEQ ID NO: 37.

In some embodiments of the invention, the isolated capsid includes a modified AAV5 capsid protein VP3, which comprises the substitutions T422A and T519S.

In some embodiments of the invention, the isolated capsid includes a modified AAV5 capsid protein VP3, which comprises the substitutions T422A and T519S, and has the amino acid sequence represented by SEQ ID NO: 38.

In some embodiments of the invention, the isolated capsid includes a modified AAV5 capsid protein VP3, which comprises the substitution T422V.

In some embodiments of the invention, the isolated capsid includes a modified AAV5 capsid protein VP3, which comprises the substitution T422V, and has the amino acid sequence represented by SEQ ID NO: 39.

In some embodiments of the invention, the isolated capsid includes a modified AAV5 capsid protein VP3, which comprises the substitutions T422V and T519S.

In some embodiments of the invention, the isolated capsid includes a modified AAV5 capsid protein VP3, which comprises the substitutions T422V and T519S, and has the amino acid sequence represented by SEQ ID NO: 40.

In one aspect, the present invention relates to an isolated nucleic acid that encodes any of the above capsids that are used for production of viral vectors based on recombinant adeno-associated virus serotype 5.

In one aspect, the present invention relates to a vector based on recombinant adeno-associated virus serotype 5 for delivering to a subject a heterologous nucleic acid sequence, comprising:
1) any of the above modified capsid proteins VP1 from adeno-associated virus serotype 5 or any of the above capsids, and
2) a heterologous nucleic acid sequence comprising regulatory sequences that promote the expression of the product encoded by the heterologous nucleic acid sequence, in the target cells.

In some embodiments of the invention, the rAAV5 vector includes a heterologous nucleic acid sequence comprising regulatory sequences that provide product expression, wherein the product of expression of the heterologous nucleic acid sequence is a therapeutic polypeptide or reporter polypeptide.

In one aspect, the present invention relates to a pharmaceutical composition for delivering a gene product to a subject in need thereof, which comprises:
a) any of the above rAAV5 vectors; and
b) a pharmaceutically acceptable excipient.

In one aspect, the present invention relates to a method for delivering a gene product to a subject in need thereof, comprising administering to the subject any of the above rAAV5 vectors or the above pharmaceutical composition.

In one aspect, the present invention relates to the use of any of the above rAAV5 vectors or the above pharmaceutical composition for treating a disease in a subject in need thereof.

In some embodiments of the use, the disease is selected from the group: blood diseases; central nervous system diseases; metabolic diseases; muscle diseases; hereditary diseases.

In one aspect, the present invention relates to a method for producing any of the above rAAV5 vectors, comprising transfecting producer cells, respectively, with any of the above nucleic acids comprising a sequence encoding a modified capsid protein VP1 from adeno-associated virus serotype 5 (AAV5), or with the above nucleic acid encoding a capsid.

### Brief description of drawings

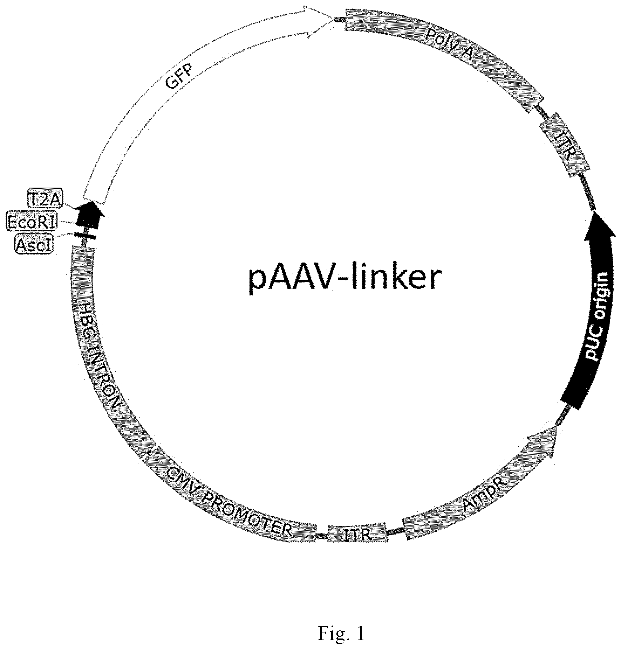

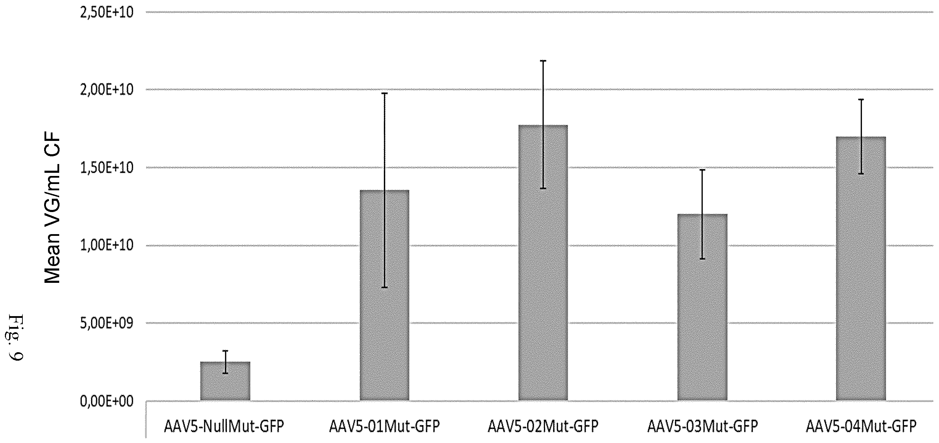

Figure 1 is a circular scheme of plasmid pAAV-linker intended for cloning the libraries of random variants of capsid gene of AAV serotype 9.
   GFP is a sequence encoding green fluorescent protein,
   PolyA is a polyadenylation signal,
   ITR is an inverted terminal repeat of adeno-associated virus,
   T2A is a sequence encoding a self-cleaving peptide from a polypeptide chain from the virus Thosea asigna,
   HBG intron is human beta-globin intron,
   CMVpromoter is human cytomegalovirus promoter,
   AmpR is a sequence of the beta-lactamase gene that provides E.coli resistance to ampicillin,
   pUC origin is a high-copy bacterial replication origin.

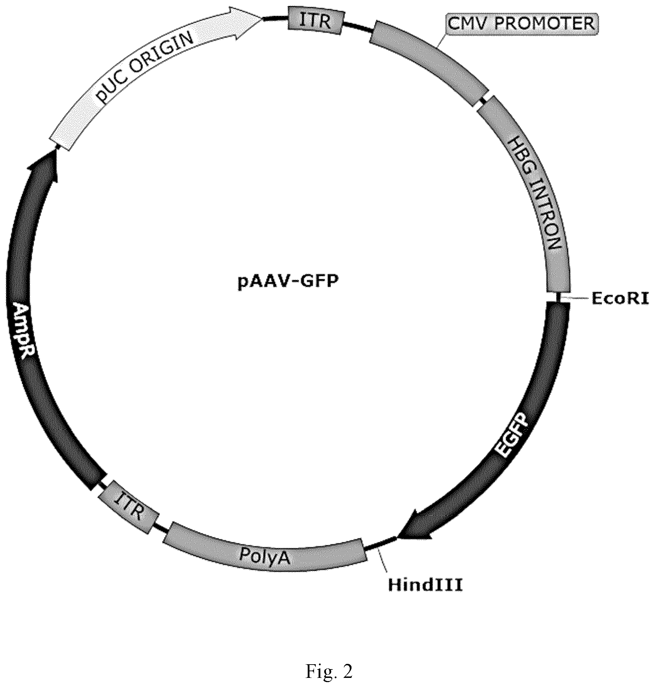

Figure 2 is a circular diagram of the plasmid pAAV-GFP.
   EGFP is a sequence encoding modified green fluorescent protein,
   PolyA is a polyadenylation signal,
   ITR is an inverted terminal repeat of adeno-associated virus,
   HBG intron is human beta-globin intron,
   CMV promoter is human cytomegalovirus promoter,
   AmpR is a sequence of the beta-lactamase gene that provides E.coli resistance to ampicillin,
   pUC origin is a high-copy bacterial replication origin.
Figure 3 is a circular scheme of plasmid pAAV-Rep intended for producing recombinant viral products of wild-type AAV serotype 5 from the library of random variants.
   AmpR is a sequence of the beta-lactamase gene that provides *E.coli* resistance to ampicillin,
   pUC origin is a high-copy bacterial replication origin,
   AAV Rep genes are a sequence encoding Rep proteins required for the virus life cycle.
Figure 4 is a circular scheme of plasmid pHelper intended for producing recombinant viral products of wild-type AAV serotype 5 from the library of random variants.
   AmpR is a beta-lactamase gene that provides resistance to ampicillin,
   Ori is a replication origin in bacteria,
   Adeno E2A is a helper adenovirus gene sequence involved in viral DNA replication,
   Adeno E4 is a helper adenovirus gene sequence involved in viral DNA replication,
   Adeno VARNA is a helper adenovirus gene sequence responsible for the translation of both early and late viral genes.

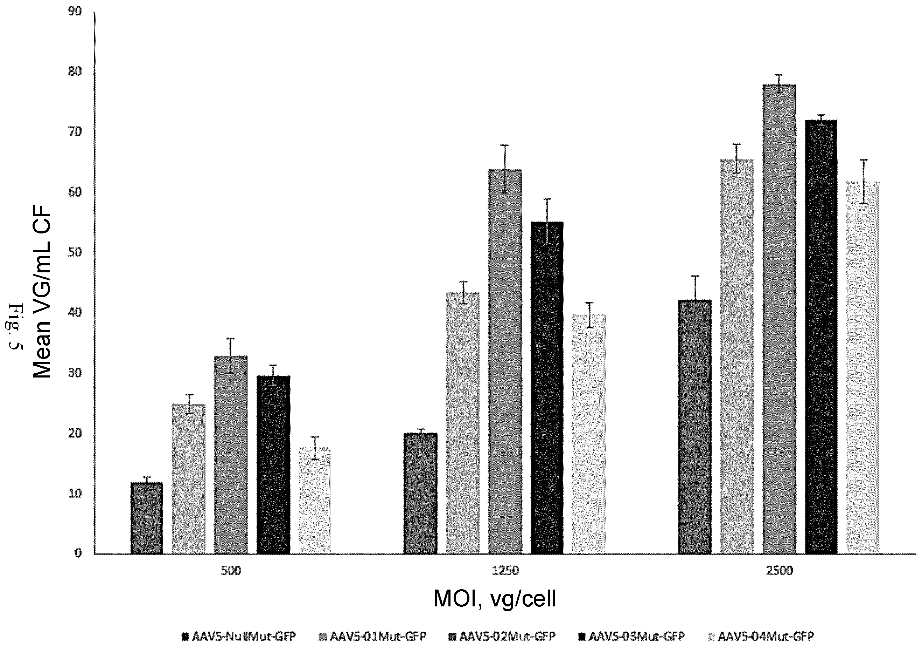

Figure 5 is a graph that shows analysis of transduction efficiency of CHO-K1-S cells with viral products based on rAAV5-GFP with mutations in the protein VP1 sequence under various MOI (viral genomes per cell).
   AAV5-01Mut-GFP refers to a vector based on modified adeno-associated virus serotype 5 (rAAV5) comprising a modified AAV5 capsid protein VP1 with the mutations S2A and T711S.
   AAV5-02Mut-GFP refers to a vector based on modified adeno-associated virus serotype 5 (rAAV5) comprising a modified AAV5 capsid protein VP1 with the mutations T614V, S2A and T711S.
   AAV5-03Mut-GFP refers to a vector based on modified adeno-associated virus serotype 5 (rAAV5) comprising a modified AAV5 capsid protein VP1 with the mutations T614A, S2A and T711S.
   AAV5-04Mut-GFP refers to a vector based on modified adeno-associated virus serotype 5 (rAAV5) comprising a modified AAV5 capsid protein VP1 with the mutations G226V, S2A and T711S.
   AAV5-NullMut-GFP refers to a vector based on modified adeno-associated virus serotype 5 (rAAV5) comprising a wild-type AAV5 capsid protein VP1.

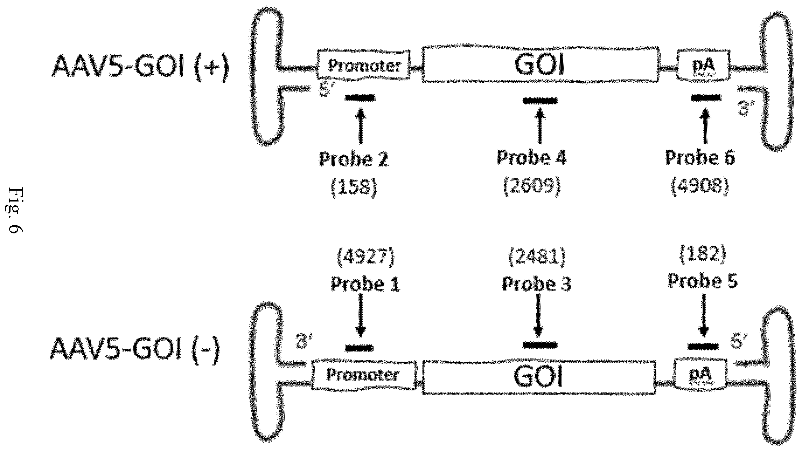

Figure 6 is a diagram showing the hybridization position of probes on the vector genome.
   Probe 1 is the detection point of a ssDNA site on the sequence directly adjacent to the 3' ITR of minus DNA strand;
   Probe 3 is the detection point of a ssDNA site located in the middle of an expression cassette on the minus DNA strand (-2400-2500 bp);
   Probe 5 is the detection point of a ssDNA site located directly adjacent to the 5' ITR of minus DNA strand.
   Probe 2 is the detection point of a ssDNA site located directly adjacent to the 5' ITR of plus DNA strand.
   Probe 4 is the detection point of a ssDNA site located in the middle of an expression cassette on the plus DNA strand (-2400-2500 bp);
   Probe 6 is the detection point of a ssDNA site located directly adjacent to the 3' ITR of plus strand. DNA.

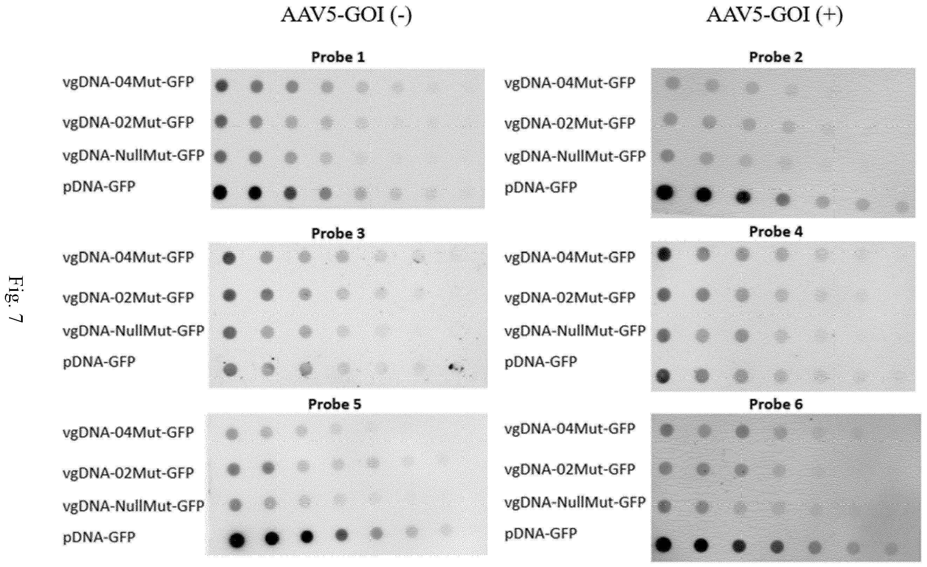

Figure 7 is Southern blot hybridization data for vector DNA.
   pDNA-GFP is a standard sample, a double serial dilution of the standard plasmid DNA pAAV-GFP.
   vgDNA-NullMut-GFP is a control sample, a double serial dilution of vector genomic DNA extracted from a control rAAV5 product with a wild-type capsid protein VP1.
   vgDNA -02Mut-GFP is a double serial dilution of vector genomic DNA extracted from rAAV5 product with capsid protein VP1 comprising the mutations S2A, T614V and T711S.
   vgDNA -04Mut-GFP is a double serial dilution of vector genomic DNA extracted from rAAV5 product with capsid protein VP1 comprising the mutations S2A, G226V and T711S.

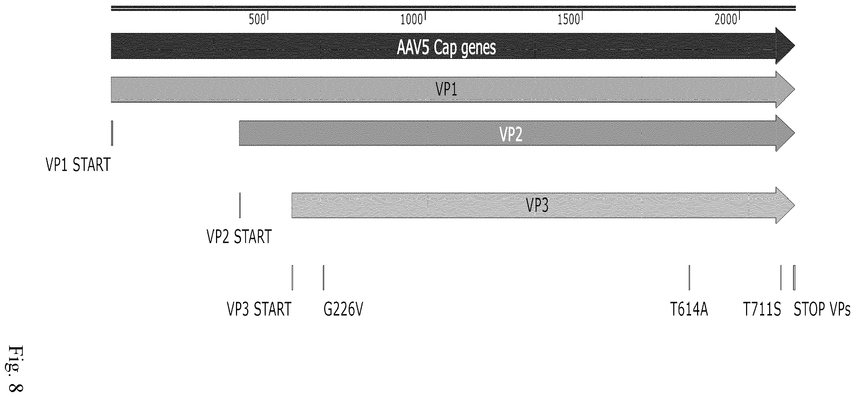

Figure 8 is a diagram showing the position of AAV5 capsid structural proteins in the amino acid sequence of the Cap gene.
   1-725 aa - VP1
   137-725 aa - VP2
   193-725 aa - VP3
Figure 9 is a graph showing analysis of generation efficiency for vectors based on modified adeno-associated virus serotype 5 (rAAV5).
   AAV5-01Mut-GFP refers to a vector based on modified adeno-associated virus serotype 5 (rAAV5) comprising a modified AAV5 capsid protein VP1 with the mutations S2A and T711S.
   AAV5-02Mut-GFP refers to a vector based on modified adeno-associated virus serotype 5 (rAAV5) comprising a modified AAV5 capsid protein VP1 with the mutations T614V, S2A and T711S.
   AAV5-03Mut-GFP refers to a vector based on modified adeno-associated virus serotype 5 (rAAV5) comprising a modified AAV5 capsid protein VP1 with the mutations T614A, S2A and T711S.
   AAV5-04Mut-GFP refers to a vector based on modified adeno-associated virus serotype 5 (rAAV5) comprising a modified AAV5 capsid protein VP1 with the mutations G226V, S2A and T711S.
   AAV5-NullMut-GFP refers to a vector based on modified adeno-associated virus serotype 5 (rAAV5) comprising a wild-type AAV5 capsid protein VP1.
   Vg/ml cf refers to viral genome count per milliliter of culture fluid.

### Definitions and general methods

Unless defined otherwise herein, all technical and scientific terms used in connection with the present invention will have the same meaning as is commonly understood by those skilled in the art.

Furthermore, unless otherwise required by context, singular terms shall include plural terms, and the plural terms shall include the singular terms. Typically, the present classification and methods of cell culture, molecular biology, immunology, microbiology, genetics, analytical chemistry, organic synthesis chemistry, medical and pharmaceutical chemistry, as well as hybridization and chemistry of protein and nucleic acids described herein are well known by those skilled and widely used in the art. Enzyme reactions and purification methods are performed according to the manufacturer's guidelines, as is common in the art, or as described herein.

"Isolated" means altered or removed from the natural state. For example, a nucleic acid or a peptide naturally present in living organisms is not "isolated" but the same nucleic acid or peptide partially or completely separated from the coexisting materials of its natural state is "isolated". An isolated nucleic acid or protein can exist substantially in purified form, or can exist in a non-native environment such as, for example, a genetically modified cell.

The terms "naturally occurring", "native", or "wild-type" are used to describe an object that can be found in nature as distinct from being artificially produced. For example, a protein or nucleotide sequence present in an organism (including a virus), which can be isolated from a source in nature and that has not been intentionally modified by a person in the laboratory, is naturally occurring.

The term "genome" refers to the complete genetic material of an organism.

As used in the present description and claims that follow, unless otherwise dictated by the context, the words "include" and "comprise", or variations thereof such as "includes", "including", "comprises", or "comprising", will be understood to imply the inclusion of a stated integer or group of integers but not the exclusion of any other integer or group of integers.

### Protein (Peptide)

As used in the present description, the terms "peptide", "polypeptide" and "protein" are used interchangeably, and they refer to a compound consisting of amino acid residues that are covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used in the present description, the term refers to both short chains, which also commonly are referred to in the art, for example, as peptides, oligopeptides and oligomers, and to longer chains, which generally are referred to in the art as proteins, of which there are many types. "Polypeptides" include, inter alia, for example, biologically active fragments, substantially homologous polypeptides, oligopeptides, homodimers, heterodimers, variants of polypeptides, modified polypeptides, derivatives, analogs, fusion proteins. The polypeptides include natural peptides, recombinant peptides, synthetic peptides, or a combination thereof.

### Nucleic acid molecules

The terms "nucleic acid", "nucleic sequence", "nucleic acid sequence", "polynucleotide", "oligonucleotide", "polynucleotide sequence" and "nucleotide sequence", used interchangeably in the present description, mean a precise sequence of nucleotides, modified or not, determining a fragment or a region of a nucleic acid, containing unnatural nucleotides or not, and being either a double-strand DNA or RNA, a single-strand DNA or RNA, or transcription products of said DNAs.

As used in the present description, polynucleotides include, as non-limiting examples, all nucleic acid sequences which are obtained by any means available in the art, including, as non-limiting examples, recombinant means, i.e. the cloning of nucleic acid sequences from a recombinant library or a cell genome, using ordinary cloning technology and PCR and the like, and by synthetic means.

It should also be included here that the present invention does not relate to nucleotide sequences in their natural chromosomal environment, i.e. in a natural state. The sequences of the present invention have been isolated and/or purified, i.e., they were sampled directly or indirectly, for example by copying, their environment having been at least partially modified. Thus, isolated nucleic acids obtained by recombinant genetics, by means, for example, of host cells, or obtained by chemical synthesis should also be mentioned here.

Unless otherwise indicated, the term nucleotide sequence encompasses its complement. Thus, a nucleic acid having a particular sequence should be understood as one which encompasses the complementary strand thereof with the complementary sequence thereof.

### Adeno-associated virus (AAV)

Viruses of the Parvoviridae family are small DNA-containing animal viruses. The Parvoviridae family may be divided into two subfamilies: the Parvovirinae, which members infect vertebrates, and the Densovirinae, which members infect insects. By 2006, there have been 11 serotypes of adeno-associated virus described (Mori, S. ET AL., 2004, "Two novel adeno-associated viruses from cynomolgus monkey: pseudotyping characterization of capsid protein", Virology, T. 330 (2): 375-83). Serotype 12 of the adeno-associated virus was described in 2008 (Michael Schmidt ET AL., Adeno-associated virus type 12 (AAV12): a novel AAV serotype with sialic acid- and heparan sulfate proteoglycan-independent transduction activity, J Virol. 2008 Feb;82(3): 1399-406. doi: 10.1128/JVI.02012-07). All of the known serotypes can infect cells from multiple tissue types. Tissue specificity is determined by the capsid protein serotype; therefore, the adeno-associated virus-based vectors are constructed by assigning the desired serotype. Further information on parvoviruses and other members of the Parvoviridae has been described in the literature (Kenneth I. Berns, «Parvoviridae: The Viruses and Their Replication», Chapter 69 in Fields Virology (3d Ed. 1996)).

The genomic organization of all known AAV serotypes is very similar. The genome of AAV is a linear, single-stranded DNA molecule that is less than about 5000 nucleotides (nt) in length. Inverted terminal repeats (ITR) flank unique coding nucleotide sequences of proteins (Rep) that are essential for the virus life cycle, as well as sequences of overlapping capsid proteins (Cap). The Cap gene encodes VP proteins (VP1, VP2 and VP3), which form a capsid, as well as AAP (protein activating adeno-associated virus (AAV) assembly (Sonntag F, Köther K, Schmidt K, et al. The assembly-activating protein promotes capsid assembly of different adeno-associated virus serotypes. J Virol. 2011;85(23):12686-12697. doi:10.1128/JVI.05359-11) and MAAP (membrane-associated accessory protein (Ogden PJ, Kelsic ED, Sinai S, Church GM. Comprehensive AAV capsid fitness landscape reveals a viral gene and enables machine-guided design. Science. 2019;366(6469):1139-1143. doi:10.1126/science.aaw2900). The AAV genome flanking sequences which are 145 nucleotides long are self-complementary and are organized such that an energetically stable intramolecular duplex forming a T-shaped hairpin may be formed. Such hairpin structures function as an origin for virus DNA replication, serving as primers for the cellular DNA polymerase complex. Following wild-type AAV (wtAAV) infection in mammalian cells, the Rep genes (e.g. Rep78 and Rep52) are expressed using the P5 promoter and the P19 promoter, respectively, and the both Rep proteins have a certain function in the replication of the viral genome. A splicing event in the Rep open reading frame (Rep ORF) results in the expression of actually four Rep proteins (e.g. Rep78, Rep68, Rep52, and Rep40). However, it has been shown that the unspliced mRNA encoding Rep78 and Rep52 proteins is sufficient for AAV vector production in mammalian cells.

### Recombinant adeno-associated virus (rAAV)-based vector

The term "vector" as used herein means a nucleic acid molecule capable of transporting another nucleic acid to which it has been linked. Furthermore, the term "vector" herein refers to a viral particle capable of transporting a nucleic acid.

As used in the present description, the term "expression" is defined as the transcription and/or translation of a particular nucleotide sequence driven by its promoter.

### Use

"Gene therapy" is the insertion of genes into subject's cells and/or tissues to treat a disease, typically hereditary diseases, in which a defective mutant allele is replaced with a functional one.

"Treat", "treating" and "treatment" refer to a method of alleviating or abrogating a biological disorder and/or at least one of its attendant symptoms. Further, references herein to "treatment" include references to curative, palliative and prophylactic treatment.

In one aspect, the subject of treatment, or patient, is a mammal, preferably a human subject. Said subject may be either male or female, of any age.

The term "disorder" means any condition that would benefit from treatment according to the present invention.

"Disease" is a state of health of a subject where the subject cannot maintain homeostasis, and where if the disease is not ameliorated then the subject's health continues to deteriorate.

The terms "subject", "patient", "individual", and the like are used interchangeably in the present description, and they refer to any animal which is amenable to the methods described in the present description. In certain non-limiting embodiments, the subject, patient or individual is a human. Said subject may be either male or female, of any age.

### Detailed description of the invention

### Isolated modified capsid protein VP1 from adeno-associated virus serotype 5 (AA V5)

In one aspect, the present invention relates to an isolated modified capsid protein VP1 from adeno-associated virus serotype 5 (AAV5) for production of viral vectors based on recombinant adeno-associated virus serotype 5 (rAAV5), which comprises a wild-type AAV5 capsid protein VP1 amino acid sequence encoded by the Cap gene with one or more substitutions that are selected from the group:
G226V,
S2A, G226V and T711S,
T614A,
S2A, T614A and T711S,
T614V,
S2A, T614V and T711S,
wherein the wild-type AAV5 capsid protein VP1 amino acid sequence has the amino acid sequence represented by

MSFVDHPPDWLEEVGEGLREFLGLEAGPPKPKPNQQHQDQARGLVLPGYNYLGPGN
GLDRGEPVNRADEVAREHDISYNEQLEAGDNPYLKYNHADAEFQEKLADDTSFGGN
LGKAVFQAKKRVLEPFGLVEEGAKTAPTGKRIDDHFPKRKKARTEEDSKPSTSSDAE
AGPSGSQQLQIPAQPASSLGADTMSAGGGGPLGDNNQGADGVGNASGDWHCDSTW
MGDRVVTKSTRTWVLPSYNNHQYREIKSGSVDGSNANAYFGYSTPWGYFDFNRFH
SHWSPRDWQRLINNYWGFRPRSLRVKIFNIQVKEVTVQDSTTTIANNLTSTVQVFTD
DDYQLPYVVGNGTEGCLPAFPPQVFTLPQYGYATLNRDNTENPTERSSFFCLEYFPS

KMLRTGNNFEFTYNFEEVPFHSSFAPSQNLFKLANPLVDQYLYRFVSTNNTGGVQFN
KNLAGRYANTYKNWFPGPMGRTQGWNLGSGVNRASVSAFATTNRMELEGASYQV
PPQPNGMTNNLQGSNTYALENTMIFNSQPANPGTTATYLEGNMLITSESETQPVNRV
AYNVGGQMATNNQSSTTAPATGTYNLQEIVPGSVWMERDVYLQGPIWAKIPETGA
HFHPSPAMGGFGLKHPPPMMLIKNTPVPGNITSFSDVPVSSFITQYSTGQVTVEMEWE
LKKENSKRWNPEIQYTNNYNDPQFVDFAPDSTGEYRTTRPIGTRYLTRPL (SEQ ID
NO: 1).

In some embodiments of the invention, the isolated modified AAV5 capsid protein VP1 comprises a substitution at position G226V.

In some embodiments of the invention, the isolated modified AAV5 capsid protein VP1 has the amino acid sequence represented by

MSFVDHPPDWLEEVGEGLREFLGLEAGPPKPKPNQQHQDQARGLVLPGYNYLGPGNGL
DRGEPVNRADEVAREHDISYNEQLEAGDNPYLKYNHADAEFQEKLADDTSFGGNLGK
AVFQAKKRVLEPFGLVEEGAKTAPTGKRIDDHFPKRKKARTEEDSKPSTSSDAEAGPSG
SQQLQIPAQPASSLGADTMSAGGGGPLGDNNQGADGVGNASGDWHCDSTWMVDRVV
TKSTRTWVLPSYNNHQYREIKSGSVDGSNANAYFGYSTPWGYFDFNRFHSHWSPRDW
QRLINNYWGFRPRSLRVKIFNIQVKEVTVQDSTTTIANNLTSTVQVFTDDDYQLPYVVG
NGTEGCLPAFPPQVFTLPQYGYATLNRDNTENPTERSSFFCLEYFPSKMLRTGNNFEFTY
NFEEVPFHSSFAPSQNLFKLANPLVDQYLYRFVSTNNTGGVQFNKNLAGRYANTYKNW
FPGPMGRTQGWNLGSGVNRASVSAFATTNRMELEGASYQVPPQPNGMTNNLQGSNTY
ALENTMIFNSQPANPGTTATYLEGNMLITSESETQPVNRVAYNVGGQMATNNQSSTTAP
ATGTYNLQEIVPGSVWMERDVYLQGPIWAKIPETGAHFHPSPAMGGFGLKHPPPMMLI
KNTPVPGNITSFSDVPVSSFITQYSTGQVTVEMEWELKKENSKRWNPEIQYTNNYNDPQ
FVDFAPDSTGEYRTTRPIGTRYLTRPL (SEQ ID NO: 3).

In some embodiments of the invention, the isolated modified AAV5 capsid protein VP1 comprises substitutions S2A, G226V and T711S.

In some embodiments of the invention, the isolated modified AAV5 capsid protein VP1 has the amino acid sequence represented by

MAFVDHPPDWLEEVGEGLREFLGLEAGPPKPKPNQQHQDQARGLVLPGYNYLGPGNG
LDRGEPVNRADEVAREHDISYNEQLEAGDNPYLKYNHADAEFQEKLADDTSFGGNLGK
AVFQAKKRVLEPFGLVEEGAKTAPTGKRIDDHFPKRKKARTEEDSKPSTSSDAEAGPSG
SQQLQIPAQPASSLGADTMSAGGGGPLGDNNQGADGVGNASGDWHCDSTWMVDRVV
TKSTRTWVLPSYNNHQYREIKSGSVDGSNANAYFGYSTPWGYFDFNRFHSHWSPRDW
QRLINNYWGFRPRSLRVKIFNIQVKEVTVQDSTTTIANNLTSTVQVFTDDDYQLPYVVG
NGTEGCLPAFPPQVFTLPQYGYATLNRDNTENPTERSSFFCLEYFPSKMLRTGNNFEFTY

NFEEVPFHSSFAPSQNLFKLANPLVDQYLYRFVSTNNTGGVQFNKNLAGRYANTYKNW
FPGPMGRTQGWNLGSGVNRASVSAFATTNRMELEGASYQVPPQPNGMTNNLQGSNTY
ALENTMIFNSQPANPGTTATYLEGNMLITSESETQPVNRVAYNVGGQMATNNQSSTTAP
ATGTYNLQEIVPGSVWMERDVYLQGPIWAKIPETGAHFHPSPAMGGFGLKHPPPMMLI
KNTPVPGNITSFSDVPVSSFITQYSTGQVTVEMEWELKKENSKRWNPEIQYTNNYNDPQ
FVDFAPDSTGEYRSTRPIGTRYLTRPL (SEQ ID NO: 4).

In some embodiments of the invention, the isolated modified AAV5 capsid protein VP1 comprises the substitution T614A.

In some embodiments of the invention, the isolated modified AAV5 capsid protein VP1 has the amino acid sequence represented by

MSFVDHPPDWLEEVGEGLREFLGLEAGPPKPKPNQQHQDQARGLVLPGYNYLGPGNGL
DRGEPVNRADEVAREHDISYNEQLEAGDNPYLKYNHADAEFQEKLADDTSFGGNLGK
AVFQAKKRVLEPFGLVEEGAKTAPTGKRIDDHFPKRKKARTEEDSKPSTSSDAEAGPSG
SQQLQIPAQPASSLGADTMSAGGGGPLGDNNQGADGVGNASGDWHCDSTWMGDRVV
TKSTRTWVLPSYNNHQYREIKSGSVDGSNANAYFGYSTPWGYFDFNRFHSHWSPRDW
QRLINNYWGFRPRSLRVKIFNIQVKEVTVQDSTTTIANNLTSTVQVFTDDDYQLPYVVG
NGTEGCLPAFPPQVFTLPQYGYATLNRDNTENPTERSSFFCLEYFPSKMLRTGNNFEFTY
NFEEVPFHSSFAPSQNLFKLANPLVDQYLYRFVSTNNTGGVQFNKNLAGRYANTYKNW
FPGPMGRTQGWNLGSGVNRASVSAFATTNRMELEGASYQVPPQPNGMTNNLQGSNTY
ALENTMIFNSQPANPGTTATYLEGNMLITSESETQPVNRVAYNVGGQMATNNQSSTTAP
ATGTYNLQEIVPGSVWMERDVYLQGPIWAKIPEAGAHFHPSPAMGGFGLKHPPPMMLI
KNTPVPGNITSFSDVPVSSFITQYSTGQVTVEMEWELKKENSKRWNPEIQYTNNYNDPQ
FVDFAPDSTGEYRTTRPIGTRYLTRPL (SEQ ID NO: 5).

In some embodiments of the invention, the isolated modified AAV5 capsid protein VP1 comprises substitutions S2A, T614A and T711S.

In some embodiments of the invention, the isolated modified AAV5 capsid protein VP1 has the amino acid sequence represented by

MAFVDHPPDWLEEVGEGLREFLGLEAGPPKPKPNQQHQDQARGLVLPGYNYLGPGNG
LDRGEPVNRADEVAREHDISYNEQLEAGDNPYLKYNHADAEFQEKLADDTSFGGNLGK
AVFQAKKRVLEPFGLVEEGAKTAPTGKRIDDHFPKRKKARTEEDSKPSTSSDAEAGPSG
SQQLQIPAQPASSLGADTMSAGGGGPLGDNNQGADGVGNASGDWHCDSTWMGDRVV
TKSTRTWVLPSYNNHQYREIKSGSVDGSNANAYFGYSTPWGYFDFNRFHSHWSPRDW
QRLINNYWGFRPRSLRVKIFNIQVKEVTVQDSTTTIANNLTSTVQVFTDDDYQLPYVVG
NGTEGCLPAFPPQVFTLPQYGYATLNRDNTENPTERSSFFCLEYFPSKMLRTGNNFEFTY
NFEEVPFHSSFAPSQNLFKLANPLVDQYLYRFVSTNNTGGVQFNKNLAGRYANTYKNW

FPGPMGRTQGWNLGSGVNRASVSAFATTNRMELEGASYQVPPQPNGMTNNLQGSNTY
ALENTMIFNSQPANPGTTATYLEGNMLITSESETQPVNRVAYNVGGQMATNNQSSTTAP
ATGTYNLQEIVPGSVWMERDVYLQGPIWAKIPEAGAHFHPSPAMGGFGLKHPPPMMLI
KNTPVPGNITSFSDVPVSSFITQYSTGQVTVEMEWELKKENSKRWNPEIQYTNNYNDPQ
FVDFAPDSTGEYRSTRPIGTRYLTRPL (SEQ ID NO: 6).

In some embodiments of the invention, the isolated modified AAV5 capsid protein VP1 comprises the substitution T614V.

In some embodiments of the invention, the isolated modified AAV5 capsid protein VP1 has the amino acid sequence represented by

MSFVDHPPDWLEEVGEGLREFLGLEAGPPKPKPNQQHQDQARGLVLPGYNYLGPGNGL
DRGEPVNRADEVAREHDISYNEQLEAGDNPYLKYNHADAEFQEKLADDTSFGGNLGK
AVFQAKKRVLEPFGLVEEGAKTAPTGKRIDDHFPKRKKARTEEDSKPSTSSDAEAGPSG
SQQLQIPAQPASSLGADTMSAGGGGPLGDNNQGADGVGNASGDWHCDSTWMGDRVV
TKSTRTWVLPSYNNHQYREIKSGSVDGSNANAYFGYSTPWGYFDFNRFHSHWSPRDW
QRLINNYWGFRPRSLRVKIFNIQVKEVTVQDSTTTIANNLTSTVQVFTDDDYQLPYVVG
NGTEGCLPAFPPQVFTLPQYGYATLNRDNTENPTERSSFFCLEYFPSKMLRTGNNFEFTY
NFEEVPFHSSFAPSQNLFKLANPLVDQYLYRFVSTNNTGGVQFNKNLAGRYANTYKNW
FPGPMGRTQGWNLGSGVNRASVSAFATTNRMELEGASYQVPPQPNGMTNNLQGSNTY
ALENTMIFNSQPANPGTTATYLEGNMLITSESETQPVNRVAYNVGGQMATNNQSSTTAP
ATGTYNLQEIVPGSVWMERDVYLQGPIWAKIPEVGAHFHPSPAMGGFGLKHPPPMMLI
KNTPVPGNITSFSDVPVSSFITQYSTGQVTVEMEWELKKENSKRWNPEIQYTNNYNDPQ
FVDFAPDSTGEYRTTRPIGTRYLTRPL (SEQ ID NO: 7).

In some embodiments of the invention, the isolated modified AAV5 capsid protein VP1 comprises substitutions S2A, T614V and T711S.

In some embodiments of the invention, the isolated modified AAV5 capsid protein VP1 has the amino acid sequence represented by

MAFVDHPPDWLEEVGEGLREFLGLEAGPPKPKPNQQHQDQARGLVLPGYNYLGPGNG
LDRGEPVNRADEVAREHDISYNEQLEAGDNPYLKYNHADAEFQEKLADDTSFGGNLGK
AVFQAKKRVLEPFGLVEEGAKTAPTGKRIDDHFPKRKKARTEEDSKPSTSSDAEAGPSG
SQQLQIPAQPASSLGADTMSAGGGGPLGDNNQGADGVGNASGDWHCDSTWMGDRVV
TKSTRTWVLPSYNNHQYREIKSGSVDGSNANAYFGYSTPWGYFDFNRFHSHWSPRDW
QRLINNYWGFRPRSLRVKIFNIQVKEVTVQDSTTTIANNLTSTVQVFTDDDYQLPYVVG
NGTEGCLPAFPPQVFTLPQYGYATLNRDNTENPTERSSFFCLEYFPSKMLRTGNNFEFTY
NFEEVPFHSSFAPSQNLFKLANPLVDQYLYRFVSTNNTGGVQFNKNLAGRYANTYKNW
FPGPMGRTQGWNLGSGVNRASVSAFATTNRMELEGASYQVPPQPNGMTNNLQGSNTY

ALENTMIFNSQPANPGTTATYLEGNMLITSESETQPVNRVAYNVGGQMATNNQSSTTAP
ATGTYNLQEIVPGSVWMERDVYLQGPIWAKIPEVGAHFHPSPAMGGFGLKHPPPMMLI
KNTPVPGNITSFSDVPVSSFITQYSTGQVTVEMEWELKKENSKRWNPEIQYTNNYNDPQ
FVDFAPDSTGEYRSTRPIGTRYLTRPL (SEQ ID NO: 8).

### Isolated modified capsid proteins VP2 and VP3 from adeno-associated virus serotype 5 (AAV5)

The "right-hand side" of a (+)-chain of genomic DNA of adeno-associated virus comprises overlapping sequences encoding three capsid proteins: VP1, VP2 and VP3. Transcription of these genes starts from a single promoter, p40. The molecular weights of the corresponding proteins are 87, 72, and 62 kDa, respectively. All of the three proteins are translated from a single mRNA. Following transcription, pre-mRNA can be spliced in two different manners, where either longer or shorter intron is excised to form mRNAs of 2300 or 2600 nucleotide long.

Thus, the introduction of mutations into the *Cap* gene will affect not only the AAV5 VP1 capsid protein, but also the AAV5 VP2 and VP3 capsid proteins.

Figure 8 is a schematic representation showing the position of structural proteins of the AAV5 capsid in the AAV Cap gene sequence:
1-725 aa - VP1
137-725 aa - VP2
193-725 aa - VP3

With the consideration of an overlapping sequence encoding the three capsid proteins, VP1, VP2 and VP3, the amino acid substitution T614A in VP1 will correspond to:
an amino acid substitution at position T478A in VP2;
an amino acid substitution at position T422A in VP3.

With the consideration of an overlapping sequence encoding the three capsid proteins, VP1, VP2 and VP3, the amino acid substitution T614V in VP1 will correspond to:
an amino acid substitution at position T478V in VP2;
an amino acid substitution at position T422V in VP3.

With the consideration of an overlapping sequence encoding the three capsid proteins, VP1, VP2 and VP3, the amino acid substitution G226V in VP1 will correspond to:
an amino acid substitution at position G90V in VP2;
an amino acid substitution at position G34V in VP3.

With the consideration of an overlapping sequence encoding the three capsid proteins, VP1, VP2 and VP3, the amino acid substitution S2A in VP1 will be absent in VP2 and VP3.

With the consideration of an overlapping sequence encoding the three capsid proteins, VP1, VP2 and VP3, the amino acid substitution T711S in VP1 will correspond to:
the amino acid substitution at position T575S in VP2;
the amino acid substitution at position T519S in VP3.

Further, the applicant considers it appropriate to specify the environment of the mutations that have been found, by indicating a short amino acid sequence including said mutations in VP1/VP2/VP3 :
For T614A in VP1 (T478A in VP2/ T422A in VP3): IPEAGAHFHP;
For T614V in VP1 (T478V in VP2/ T422V in VP3): IPEVGAHFHP;
For G226V in VP1 (G90V in VP2/ G34V in VP3): TWMVDRV;
For S2A in VP1 (missing in VP2 and in VP3): MAFVDHP;
For T711S in VP1 (T575S in VP2/ T519S in VP3): EYRSTRP.

In some embodiments of the invention, the amino acid sequence of the wild-type AAV5 capsid protein VP2 has the amino acid sequence represented by SEQ ID NO: 17.

In some embodiments of the invention, the amino acid sequence of the modified AAV5 capsid protein VP2 comprises the substitution G90V.

In some embodiments of the invention, the amino acid sequence of the modified AAV5 capsid protein VP2 comprises the substitution G90V and has the amino acid sequence represented by SEQ ID NO: 19.

In some embodiments of the invention, the amino acid sequence of the modified AAV5 capsid protein VP2 comprises the substitutions G90V and T575S.

In some embodiments of the invention, the amino acid sequence of the modified AAV5 capsid protein VP2 comprises the substitutions G90V and T575S and has the amino acid sequence represented by SEQ ID NO: 20.

In some embodiments of the invention, the amino acid sequence of the modified AAV5 capsid protein VP2 comprises the substitution T478A.

In some embodiments of the invention, the amino acid sequence of the modified AAV5 capsid protein VP2 comprises T478A and has the amino acid sequence represented by SEQ ID NO: 21.

In some embodiments of the invention, the amino acid sequence of the modified AAV5 capsid protein VP2 comprises the substitutions T478A and T575S.

In some embodiments of the invention, the amino acid sequence of the modified AAV5 capsid protein VP2 comprises the substitutions T478A and T575S and has the amino acid sequence represented by SEQ ID NO: 22.

In some embodiments of the invention, the amino acid sequence of the modified AAV5 capsid protein VP2 comprises the substitution T478V.

In some embodiments of the invention, the amino acid sequence of the modified AAV5 capsid protein VP2 comprises the substitution T478V and has the amino acid sequence represented by SEQ ID NO: 23.

In some embodiments of the invention, the amino acid sequence of the modified AAV5 capsid protein VP2 comprises the substitutions T478V and T575S.

In some embodiments of the invention, the amino acid sequence of the modified AAV5 capsid protein VP2 comprises the substitutions T478V and T575S and has the amino acid sequence represented by SEQ ID NO: 24.

In some embodiments of the invention, the amino acid sequence of the wild-type AAV5 capsid protein VP3 has the amino acid sequence represented by SEQ ID NO: 33.

In some embodiments of the invention, the amino acid sequence of the modified AAV5 capsid protein VP3 comprises the substitution G34V.

In some embodiments of the invention, the amino acid sequence of the modified AAV5 capsid protein VP3 comprises the substitution G34V and has the amino acid sequence represented by SEQ ID NO: 35.

In some embodiments of the invention, the amino acid sequence of the modified AAV5 capsid protein VP3 comprises the substitutions G34V and T519S.

In some embodiments of the invention, the amino acid sequence of the modified AAV5 capsid protein VP3 comprises the substitutions G34V and T519S and has the amino acid sequence represented by SEQ ID NO: 36.

In some embodiments of the invention, the amino acid sequence of the modified AAV5 capsid protein VP3 comprises the substitution T422A.

In some embodiments of the invention, the amino acid sequence of the modified AAV5 capsid protein VP3 comprises the substitution T422A and has the amino acid sequence represented by SEQ ID NO: 37.

In some embodiments of the invention, the amino acid sequence of the modified AAV5 capsid protein VP3 comprises the substitutions T422A and T519S.

In some embodiments of the invention, the amino acid sequence of the modified AAV5 capsid protein VP3 comprises the substitutions T422A and T519S and has the amino acid sequence represented by SEQ ID NO: 38.

In some embodiments of the invention, the amino acid sequence of the modified AAV5 capsid protein VP3 comprises the substitution T422V.

In some embodiments of the invention, the amino acid sequence of the modified AAV5 capsid protein VP3 comprises the substitution T422V and has the amino acid sequence represented by SEQ ID NO: 39.

In some embodiments of the invention, the amino acid sequence of the modified AAV5 capsid protein VP3 comprises the substitutions T422V and T519S.

In some embodiments of the invention, the amino acid sequence of the modified AAV5 capsid protein VP3 comprises the substitutions T422V and T519S and has the amino acid sequence represented by SEQ ID NO: 40.

### Capsid

In one aspect, the present invention relates to an isolated capsid for production of viral vectors based on recombinant adeno-associated virus serotype 5, comprising any of the above modified capsid proteins VP1 from adeno-associated virus serotype 5.

In some embodiments of the invention, the isolated capsid includes any of the above modified capsid proteins VP1 from adeno-associated virus serotype 5, AAV5 capsid protein VP2 or a modified variant thereof, and AAV5 capsid protein VP3 or a modified variant thereof.

In some embodiments of the invention, the isolated capsid includes a wild-type AAV5 capsid protein VP2.

In some embodiments of the invention, the isolated capsid includes a wild-type AAV5 capsid protein VP2, which has the amino acid sequence represented by SEQ ID NO: 17.

In some embodiments of the invention, the isolated capsid includes a modified capsid protein VP2 from adeno-associated virus serotype 5.

In some embodiments of the invention, the isolated capsid includes a modified AAV5 capsid protein VP2, which comprises the substitution G90V.

In some embodiments of the invention, the isolated capsid includes a modified AAV5 capsid protein VP2, which comprises the substitution G90V, and has the amino acid sequence represented by SEQ ID NO: 19.

In some embodiments of the invention, the isolated capsid includes a modified AAV5 capsid protein VP2, which comprises the substitutions G90V and T575S.

In some embodiments of the invention, the isolated capsid includes a modified AAV5 capsid protein VP2, which comprises the substitutions G90V and T575S, and has the amino acid sequence represented by SEQ ID NO: 20.

In some embodiments of the invention, the isolated capsid includes a modified AAV5 capsid protein VP2, which comprises the substitution T478A.

In some embodiments of the invention, the isolated capsid includes a modified AAV5 capsid protein VP2, which comprises the substitution T478A, and has the amino acid sequence represented by SEQ ID NO: 21.

In some embodiments of the invention, the isolated capsid includes a modified AAV5 capsid protein VP2, which comprises the substitutions T478A and T575S.

In some embodiments of the invention, the isolated capsid includes a modified AAV5 protein, which comprises the substitutions T478A and T575S, and has the amino acid sequence represented by SEQ ID NO: 22.

In some embodiments of the invention, the isolated capsid includes a modified AAV5 capsid protein VP2, which comprises the substitution T478V.

In some embodiments of the invention, the isolated capsid includes a modified AAV5 capsid protein VP2, which comprises the substitution T478V, and has the amino acid sequence represented by SEQ ID NO: 23.

In some embodiments of the invention, the isolated capsid includes a modified AAV5 capsid protein VP2, which comprises the substitutions T478V and T575S.

In some embodiments of the invention, the isolated capsid includes a modified AAV5 capsid protein VP2, which comprises the substitutions T478V and T575S, and has the amino acid sequence represented by SEQ ID NO: 24.

In some embodiments of the invention, the isolated capsid includes a wild-type AAV5 capsid protein VP3.

In some embodiments of the invention, the isolated capsid includes a wild-type AAV5 capsid protein VP3, which has the amino acid sequence represented by SEQ ID NO: 33.

In some embodiments of the invention, the isolated capsid includes a modified capsid protein VP3 from adeno-associated virus serotype 5.

In some embodiments of the invention, the isolated capsid includes a modified AAV5 capsid protein VP3, which comprises the substitution G34V.

In some embodiments of the invention, the isolated capsid includes a modified AAV5 capsid protein VP3, which comprises the substitution G34V, and has the amino acid sequence represented by SEQ ID NO: 35.

In some embodiments of the invention, the isolated capsid includes a modified AAV5 capsid protein VP3, which comprises the substitutions G34V and T519S.

In some embodiments of the invention, the isolated capsid includes a modified AAV5 capsid protein VP3, which comprises the substitutions G34V and T519S, and has the amino acid sequence represented by SEQ ID NO: 36.

In some embodiments of the invention, the isolated capsid includes a modified AAV5 capsid protein VP3, which comprises the substitution T422A.

In some embodiments of the invention, the isolated capsid includes a modified AAV5 capsid protein VP3, which comprises the substitution T422A, and has the amino acid sequence represented by SEQ ID NO: 37.

In some embodiments of the invention, the isolated capsid includes a modified AAV5 capsid protein VP3, which comprises the substitutions T422A and T519S.

In some embodiments of the invention, the isolated capsid includes a modified AAV5 capsid protein VP3, which comprises the substitutions T422A and T519S, and has the amino acid sequence represented by SEQ ID NO: 38.

In some embodiments of the invention, the isolated capsid includes a modified AAV5 capsid protein VP3, which comprises the substitution T422V.

In some embodiments of the invention, the isolated capsid includes a modified AAV5 capsid protein VP3, which comprises the substitution T422V, and has the amino acid sequence represented by SEQ ID NO: 39.

In some embodiments of the invention, the isolated capsid includes a modified AAV5 capsid protein VP3, which comprises the substitutions T422V and T519S.

In some embodiments of the invention, the isolated capsid includes a modified AAV5 capsid protein VP3, which comprises the substitutions T422V and T519S, and has the amino acid sequence represented by SEQ ID NO: 40.

### Isolated nucleic acid

In one aspect, the present invention relates to an isolated nucleic acid that encodes any of the above modified capsid proteins VP1 from adeno-associated virus serotype 5 that are used for production of viral vectors based on recombinant adeno-associated virus serotype 5.

In some embodiments of the invention, the isolated nucleic acid encodes a modified capsid protein VP1 from adeno-associated virus serotype 5 with the amino acid substitution G226V, which is represented by a nucleic sequence with SEQ ID NO: 11 or by any other sequence encoding the respective amino acid sequence.

"Other sequence encoding the corresponding amino acid sequence of the modified capsid protein VP1 from adeno-associated virus serotype 5 (AAV5) with the amino acid substitution G226V" means a nucleic sequence that is alternative to the nucleic sequence with SEQ ID NO: 11, as, due to the degeneracy of genetic code, a wide range of different DNA sequences can encode the amino acid sequence disclosed herein as SEQ ID NO: 3. It is well within the skill of a person trained in the art to create these alternative DNA sequences encoding the same amino acid sequences. Such variant DNA sequences are within the scope of the present invention.

In some embodiments of the invention, the isolated nucleic acid encodes a modified capsid protein VP1 from adeno-associated virus serotype 5 with the amino acid substitutions S2A, G226V and T711S and is represented by a nucleic sequence with SEQ ID NO: 12 or by any other sequence encoding the respective amino acid sequence.

"Other sequence encoding the corresponding amino acid sequence of the modified capsid protein VP1 from adeno-associated virus serotype 5 (AAV5) with the amino acid substitutions S2A, G226V and T711S" means a nucleic sequence that is alternative to the nucleic sequence with SEQ ID NO: 12, as, due to the degeneracy of genetic code, a wide range of different DNA sequences can encode the amino acid sequence disclosed herein as SEQ ID NO: 4. It is well within the skill of a person trained in the art to create these alternative DNA sequences encoding the same amino acid sequences. Such variant DNA sequences are within the scope of the present invention.

In some embodiments of the invention, the isolated nucleic acid encodes a modified capsid protein VP1 from adeno-associated virus serotype 5 with the amino acid substitution T614A and is represented by a nucleic sequence with SEQ ID NO: 13 or by any other sequence encoding the respective amino acid sequence.

"Other sequence encoding the corresponding amino acid sequence of the modified capsid protein VP1 from adeno-associated virus serotype 5 (AAV5) with the amino acid substitution T614A" means a nucleic sequence that is alternative to the nucleic sequence with SEQ ID NO: 13, as, due to the degeneracy of genetic code, a wide range of different DNA sequences can encode the amino acid sequence disclosed herein as SEQ ID NO: 5. It is well within the skill of a person trained in the art to create these alternative DNA sequences encoding the same amino acid sequences. Such variant DNA sequences are within the scope of the present invention.

In some embodiments of the invention, the isolated nucleic acid encodes a modified capsid protein VP1 from adeno-associated virus serotype 5 with the amino acid substitutions S2A, T614A and T711S and is represented by a nucleic sequence with SEQ ID NO: 14 or by any other sequence encoding the respective amino acid sequence.

"Other sequence encoding the corresponding amino acid sequence of the modified capsid protein VP1 from adeno-associated virus serotype 5 (AAV5) with the amino acid substitutions S2A, T614A and T711S" means a nucleic sequence that is alternative to the nucleic sequence with SEQ ID NO: 14, as, due to the degeneracy of genetic code, a wide range of different DNA sequences can encode the amino acid sequence disclosed herein as SEQ ID NO: 6. It is well within the skill of a person trained in the art to create these alternative DNA sequences encoding the same amino acid sequences. Such variant DNA sequences are within the scope of the present invention.

In some embodiments of the invention, the isolated nucleic acid encodes a modified capsid protein VP1 from adeno-associated virus serotype 5 with the amino acid substitution T614V and is represented by a nucleic sequence with SEQ ID NO: 15 or by any other sequence encoding the respective amino acid sequence.

"Other sequence encoding the corresponding amino acid sequence of the modified capsid protein VP1 from adeno-associated virus serotype 5 (AAV5) with the amino acid substitution T614V" means a nucleic sequence that is alternative to the nucleic sequence with SEQ ID NO: 15, as, due to the degeneracy of genetic code, a wide range of different DNA sequences can encode the amino acid sequence disclosed herein as SEQ ID NO: 7. It is well within the skill of a person trained in the art to create these alternative DNA sequences encoding the same amino acid sequences. Such variant DNA sequences are within the scope of the present invention.

In some embodiments of the invention, the isolated nucleic acid encodes a modified capsid protein VP1 from adeno-associated virus serotype 5 with the amino acid substitutions S2A, T614V and T711S and is represented by a nucleic sequence with SEQ ID NO: 16 or by any other sequence encoding the respective amino acid sequence.

"Other sequence encoding the corresponding amino acid sequence of the modified capsid protein VP1 from adeno-associated virus serotype 5 (AAV5) with the amino acid substitutions S2A, T614V and T711S" means a nucleic sequence that is alternative to the nucleic sequence with SEQ ID NO: 16, as, due to the degeneracy of genetic code, a wide range of different DNA sequences can encode the amino acid sequence disclosed herein as SEQ ID NO: 8. It is well within the skill of a person trained in the art to create these alternative DNA sequences encoding the same amino acid sequences. Such variant DNA sequences are within the scope of the present invention.

In one aspect, the present invention relates to an isolated nucleic acid encoding the above modified capsid protein VP2 from adeno-associated virus serotype 5 (AAV5).

In some embodiments of the invention, the isolated nucleic acid encodes a modified capsid protein VP2 from adeno-associated virus serotype 5 (AAV5) with the amino acid substitution G90V and is represented by a nucleic sequence with SEQ ID NO: 27 or by any other sequence encoding the respective amino acid sequence.

"Other sequence encoding the corresponding amino acid sequence of the modified capsid protein VP2 from adeno-associated virus serotype 5 (AAV5) with the amino acid substitution G90V" means a nucleic sequence that is alternative to the nucleic sequence with SEQ ID NO: 27, as, due to the degeneracy of genetic code, a wide range of different DNA sequences can encode the amino acid sequence disclosed herein as SEQ ID NO: 19. It is well within the skill of a person trained in the art to create these alternative DNA sequences encoding the same amino acid sequences. Such variant DNA sequences are within the scope of the present invention.

In some embodiments of the invention, the isolated nucleic acid encodes a modified capsid protein VP2 from adeno-associated virus serotype 5 (AAV5) with the amino acid substitutions G90V and T575S and is represented by a nucleic sequence with SEQ ID NO: 28 or by any other sequence encoding the respective amino acid sequence.

"Other sequence encoding the corresponding amino acid sequence of the modified capsid protein VP2 from adeno-associated virus serotype 5 (AAV5) with the amino acid substitutions G90V and T575S" means a nucleic sequence that is alternative to the nucleic sequence with SEQ ID NO: 28, as, due to the degeneracy of genetic code, a wide range of different DNA sequences can encode the amino acid sequence disclosed herein as SEQ ID NO: 20. It is well within the skill of a person trained in the art to create these alternative DNA sequences encoding the same amino acid sequences. Such variant DNA sequences are within the scope of the present invention.

In some embodiments of the invention, the isolated nucleic acid encodes a modified capsid protein VP2 from adeno-associated virus serotype 5 (AAV5) with the amino acid substitution T478A and is represented by a nucleic sequence with SEQ ID NO: 29 or by any other sequence encoding the respective amino acid sequence.

"Other sequence encoding the corresponding amino acid sequence of the modified capsid protein VP2 from adeno-associated virus serotype 5 (AAV5) with the amino acid substitution T478A" means a nucleic sequence that is alternative to the nucleic sequence with SEQ ID NO: 29, as, due to the degeneracy of genetic code, a wide range of different DNA sequences can encode the amino acid sequence disclosed herein as SEQ ID NO: 21. It is well within the skill of a person trained in the art to create these alternative DNA sequences encoding the same amino acid sequences. Such variant DNA sequences are within the scope of the present invention.

In some embodiments of the invention, the isolated nucleic acid encodes a modified capsid protein VP2 from adeno-associated virus serotype 5 (AAV5) with the amino acid substitutions T478A and T575S and is represented by a nucleic sequence with SEQ ID NO: 30 or by any other sequence encoding the respective amino acid sequence.

"Other sequence encoding the corresponding amino acid sequence of the modified capsid protein VP2 from adeno-associated virus serotype 5 (AAV5) with the amino acid substitutions T478A and T575S" means a nucleic sequence that is alternative to the nucleic sequence with SEQ ID NO: 30, as, due to the degeneracy of genetic code, a wide range of different DNA sequences can encode the amino acid sequence disclosed herein as SEQ ID NO: 22. It is well within the skill of a person trained in the art to create these alternative DNA sequences encoding the same amino acid sequences. Such variant DNA sequences are within the scope of the present invention.

In some embodiments of the invention, the isolated nucleic acid encodes a modified capsid protein VP2 from adeno-associated virus serotype 5 (AAV5) with the amino acid substitution T478V and is represented by a nucleic sequence with SEQ ID NO: 31 or by any other sequence encoding the respective amino acid sequence.

"Other sequence encoding the corresponding amino acid sequence of the modified capsid protein VP2 from adeno-associated virus serotype 5 (AAV5) with the amino acid substitution T478V" means a nucleic sequence that is alternative to the nucleic sequence with SEQ ID NO: 31, as, due to the degeneracy of genetic code, a wide range of different DNA sequences can encode the amino acid sequence disclosed herein as SEQ ID NO: 23. It is well within the skill of a person trained in the art to create these alternative DNA sequences encoding the same amino acid sequences. Such variant DNA sequences are within the scope of the present invention.

In some embodiments of the invention, the isolated nucleic acid encodes a modified capsid protein VP2 from adeno-associated virus serotype 5 (AAV5) with the amino acid substitutions T478V and T575S and is represented by a nucleic sequence with SEQ ID NO: 32 or by any other sequence encoding the respective amino acid sequence.

"Other sequence encoding the corresponding amino acid sequence of the modified capsid protein VP2 from adeno-associated virus serotype 5 (AAV5) with the amino acid substitutions T478V and T575S" means a nucleic sequence that is alternative to the nucleic sequence with SEQ ID NO: 32, as, due to the degeneracy of genetic code, a wide range of different DNA sequences can encode the amino acid sequence disclosed herein as SEQ ID NO: 24. It is well within the skill of a person trained in the art to create these alternative DNA sequences encoding the same amino acid sequences. Such variant DNA sequences are within the scope of the present invention.

In one aspect, the present invention relates to an isolated nucleic acid encoding the above modified capsid protein VP3 from adeno-associated virus serotype 5 (AAV5).

In some embodiments of the invention, the isolated nucleic acid encodes a modified capsid protein VP3 from adeno-associated virus serotype 5 (AAV5) with the amino acid substitution G34V and is represented by a nucleic sequence with SEQ ID NO: 43 or by any other sequence encoding the respective amino acid sequence.

"Other sequence encoding the corresponding amino acid sequence of the modified capsid protein VP3 from adeno-associated virus serotype 5 (AAV5) with the amino acid substitution G34V" means a nucleic sequence that is alternative to the nucleic sequence with SEQ ID NO: 43, as, due to the degeneracy of genetic code, a wide range of different DNA sequences can encode the amino acid sequence disclosed herein as SEQ ID NO: 35. It is well within the skill of a person trained in the art to create these alternative DNA sequences encoding the same amino acid sequences. Such variant DNA sequences are within the scope of the present invention.

In some embodiments of the invention, the isolated nucleic acid encodes a modified capsid protein VP3 from adeno-associated virus serotype 5 (AAV5) with the amino acid substitutions G34V and T519S and is represented by a nucleic sequence with SEQ ID NO: 44 or by any other sequence encoding the respective amino acid sequence.

"Other sequence encoding the corresponding amino acid sequence of the modified capsid protein VP3 from adeno-associated virus serotype 5 (AAV5) with the amino acid substitutions G34V and T519S" means a nucleic sequence that is alternative to the nucleic sequence with SEQ ID NO: 44, as, due to the degeneracy of genetic code, a wide range of different DNA sequences can encode the amino acid sequence disclosed herein as SEQ ID NO: 36. It is well within the skill of a person trained in the art to create these alternative DNA sequences encoding the same amino acid sequences. Such variant DNA sequences are within the scope of the present invention.

In some embodiments of the invention, the isolated nucleic acid encodes a modified capsid protein VP3 from adeno-associated virus serotype 5 (AAV5) with the amino acid substitution T422A and is represented by a nucleic sequence with SEQ ID NO: 45 or by any other sequence encoding the respective amino acid sequence.

"Other sequence encoding the corresponding amino acid sequence of the modified capsid protein VP3 from adeno-associated virus serotype 5 (AAV5) with the amino acid substitution T422A" means a nucleic sequence that is alternative to the nucleic sequence with SEQ ID NO: 45, as, due to the degeneracy of genetic code, a wide range of different DNA sequences can encode the amino acid sequence disclosed herein as SEQ ID NO: 37. It is well within the skill of a person trained in the art to create these alternative DNA sequences encoding the same amino acid sequences. Such variant DNA sequences are within the scope of the present invention.

In some embodiments of the invention, the isolated nucleic acid encodes a modified capsid protein VP3 from adeno-associated virus serotype 5 (AAV5) with the amino acid substitutions T422A and T519S and is represented by a nucleic sequence with SEQ ID NO: 46 or by any other sequence encoding the respective amino acid sequence.

"Other sequence encoding the corresponding amino acid sequence of the modified capsid protein VP3 from adeno-associated virus serotype 5 (AAV5) with the amino acid substitutions T422A and T519S" means a nucleic sequence that is alternative to the nucleic sequence with SEQ ID NO: 46, as, due to the degeneracy of genetic code, a wide range of different DNA sequences can encode the amino acid sequence disclosed herein as SEQ ID NO: 38. It is well within the skill of a person trained in the art to create these alternative DNA sequences encoding the same amino acid sequences. Such variant DNA sequences are within the scope of the present invention.

In some embodiments of the invention, the isolated nucleic acid encodes a modified capsid protein VP3 from adeno-associated virus serotype 5 (AAV5) with the amino acid substitution T422V and is represented by a nucleic sequence with SEQ ID NO: 47 or by any other sequence encoding the respective amino acid sequence.

"Other sequence encoding the corresponding amino acid sequence of the modified capsid protein VP3 from adeno-associated virus serotype 5 (AAV5) with the amino acid substitution T422V" means a nucleic sequence that is alternative to the nucleic sequence with SEQ ID NO: 47, as, due to the degeneracy of genetic code, a wide range of different DNA sequences can encode the amino acid sequence disclosed herein as SEQ ID NO: 39. It is well within the skill of a person trained in the art to create these alternative DNA sequences encoding the same amino acid sequences. Such variant DNA sequences are within the scope of the present invention.

In some embodiments of the invention, the isolated nucleic acid encodes a modified capsid protein VP3 from adeno-associated virus serotype 5 (AAV5) with the amino acid substitutions T422V and T519S and is represented by a nucleic sequence with SEQ ID NO: 48 or by any other sequence encoding the respective amino acid sequence.

"Other sequence encoding the corresponding amino acid sequence of the modified capsid protein VP3 from adeno-associated virus serotype 5 (AAV5) with the amino acid substitutions T422V and T519S" means a nucleic sequence that is alternative to the nucleic sequence with SEQ ID NO: 48, as, due to the degeneracy of genetic code, a wide range of different DNA sequences can encode the amino acid sequence disclosed herein as SEQ ID NO: 40. It is well within the skill of a person trained in the art to create these alternative DNA sequences encoding the same amino acid sequences. Such variant DNA sequences are within the scope of the present invention.

In some embodiments, the isolated nucleic acid encoding the above capsid includes any of the above nucleic acid sequences or combinations thereof.

### Vector based on recombinant adeno-associated virus serotype 5 (rAA V5)

In one aspect, the present invention relates to a vector based on recombinant adeno-associated virus serotype 5 (rAAV5) for delivering to a subject a heterologous nucleic acid sequence, comprising:
1) any of the above modified capsid proteins VP1 from adeno-associated virus serotype 5 (AAV5) or any of the above capsids, and
2) a heterologous nucleic acid sequence comprising regulatory sequences that promote the expression of the product encoded by the heterologous nucleic acid sequence, in the target cells.

In one aspect, the present invention relates to a vector based on recombinant adeno-associated virus serotype 5 (rAAV5) for delivering to a subject a heterologous nucleic acid sequence, comprising:
1) any of the above modified capsid proteins VP1 from adeno-associated virus serotype 5 (AAV5), and
2) a heterologous nucleic acid sequence comprising regulatory sequences that promote the expression of the product encoded by the heterologous nucleic acid sequence, in the target cells.

In one aspect, the present invention relates to a vector based on recombinant adeno-associated virus serotype 5 (rAAV5) for delivering to a subject a heterologous nucleic acid sequence, comprising:
1) any of the above capsids, and
2) a heterologous nucleic acid sequence comprising regulatory sequences that promote the expression of the product encoded by the heterologous nucleic acid sequence, in the target cells.

The terms "vector based on recombinant adeno-associated virus serotype 5", "recombinant virus based on AAV5", "virus-like particle based on AAV5", "recombinant viral AAV5 strain", "recombinant AAV5 vector" or "vector based on rAAV5" as used in the present description have the same meaning.

In some embodiments of the invention, the rAAV5 vector includes a heterologous nucleic acid sequence comprising regulatory sequences that provide product expression, wherein the product of expression of the heterologous nucleic acid sequence is a therapeutic polypeptide or reporter polypeptide.

The rAAV vector according to the invention does not comprise nucleotide sequences of genes encoding sequences of proteins (Rep) that are essential for the virus life cycle, as well as sequences of overlapping capsid proteins (Cap).

The capsid is characterized in detail in the above section of the description.

"Regulatory sequences that provide the expression of product encoded by the heterologous nucleic acid sequence in target cells", as used in the present invention, mean polynucleotide sequences that are necessary to influence the expression and processing of coding sequences to which they are cloned. Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (i.e. Kozak consensus sequence); sequences that enhance protein stability; and when desired, sequences that enhance protein secretion. The nature of such control sequences differs depending upon the host organism; in prokaryotes, such control sequences generally include the promoter of ribosome binding site, and transcription termination sequences; in eukaryotes, typically, such control sequences include promoters and transcription termination sequences. The term "regulatory sequences" includes at least all components, the presence of which is essential for expression and processing, and can also include additional components, the presence of which is advantageous, for example, leader peptide sequences.

As used in the present description, the term "promoter" refers to a nucleic acid fragment that controls the transcription of one or more coding sequences and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art that directly or indirectly regulate the level of transcription with said promoter. A "constitutive" promoter is a promoter that is active in most tissues under typical physiological and developmental conditions. An "inducible" promoter is a promoter that is physiologically or developmentally regulated, e.g. under the influence of a chemical inducer. A "tissue specific" promoter is only active in specific types of tissues or cells.

Promoters that are used for high-level production of polypeptides in eukaryotic cells and, in particular, in mammalian cells, should be strong and preferably active in a wide range of cell types. Strong constitutive promoters which are capable of driving expression in many cell types are well known in the art and, therefore, it is not herein necessary to describe them in detail. In accordance with the idea of the present invention, it is preferable to use the cytomegalovirus (CMV) promoter. A promoter or promoter/enhancer derived from the immediate early (IE) region of human cytomegalovirus (hCMV) is particularly suitable as a promoter for the rAAV5 vector of the present invention. Human cytomegalovirus (hCMV) immediate early (IE) region and functional expression-inducing and/or functional expression-enhancing fragments derived therefrom, for example, have been disclosed in EP0173177 and EP0323997, and are well known in the art. Thus, several fragments of the hCMV immediate early (IE) region may be used as a promoter and/or promoter/enhancer.

The terms "enhancers" or "enhancer" as used herein may refer to a DNA sequence that is located adjacent to the DNA sequence that encodes a recombinant product. Enhancer elements are typically located in a 5' direction from a promoter element or can be located downstream of or within a coding DNA sequence (e.g. a DNA sequence transcribed or translated into a recombinant product or products). Hence, an enhancer element may be located 100 base pairs, 200 base pairs, or 300 or more base pairs upstream of a DNA sequence that encodes a recombinant product, or downstream of said sequence. Enhancer elements may increase the amount of a recombinant product being expressed from a DNA sequence above the level of expression associated with a single promoter element. Multiple enhancer elements are readily available to those of ordinary skill in the art.

In some embodiments of the invention, the heterologous nucleic acid sequence comprising regulatory sequences providing the expression of a product encoded by the heterologous nucleic acid sequence in target cells may include the following elements in the 5'-end to 3'-end direction:
a left-hand (first) ITR (inverted terminal repeats);
A CMV (cytomegalovirus) enhancer;
A CMV (cytomegalovirus) promoter;
an intron of the hBG1 gene (hemoglobin subunit gamma 1 gene);
a nucleic acid encoding the product;
an hGH1 polyadenylation signal (human growth hormone gene polyadenylation signal);
a right-hand (second) ITR.

In some embodiments, the nucleic acid encoding the product (transgene) is at least one gene encoding a protein. In some embodiments, the transgene encodes at least one small inhibitory nucleic acid. In some embodiments, the transgene encodes at least one reporter molecule. In some embodiments, the small inhibitory nucleic acid is miRNA. In some embodiments, the small inhibitory nucleic acid is sponge miRNA or TuD-RNA, which inhibits the activity of at least one miRNA in an animal. In some embodiments, miRNA is expressed in a cell of the target tissue. In some embodiments, the target tissue is the tissue of the liver, central nervous system (CNS), eyes, gastrointestinal tract, respiratory tract, breast, pancreas, urinary tract or uterine tissue.

In some embodiments, the rAAV5 vector has a product of expression of the heterologous nucleic acid sequence, which is a therapeutic polypeptide or a reporter polypeptide.

In some embodiments, the rAAV5 vector comprises a heterologous nucleic acid sequence encoding a product that is a therapeutic polypeptide, wherein the therapeutic polypeptide is a coagulation factor selected from the group consisting of Factor VIII, Factor IX, or a functional variant thereof.

In some embodiments, the rAAV5 vector comprises a heterologous nucleic acid sequence encoding a product that is Factor VIII or a functional variant thereof.

In some embodiments, the rAAV5 vector comprises a heterologous nucleic acid sequence encoding a product that is Factor IX or a functional variant thereof.

In some embodiments, the rAAV5 vector comprises a heterologous nucleic acid sequence encoding a product that is the SMN1 protein (survival motor neuron protein)

In some embodiments, the rAAV5 vector comprises a heterologous nucleic acid sequence encoding a product that is the RBD-S polypeptide (recombinant receptor-binding domain of S glycoprotein) of SARS-cov2 (severe acute respiratory syndrome coronavirus 2).

### Pharmaceutical composition

In one aspect, the present invention relates to a pharmaceutical composition for delivering a gene product to a subject in need thereof, which comprises:
a) any of the above rAAV5 vectors; and
b) a pharmaceutically acceptable excipient.

In some embodiments, the pharmaceutical composition is used for delivering a gene product to a human in need thereof.

In particular embodiments, the present invention relates to a pharmaceutical composition comprising the rAAV5 viral particle of the invention in a pharmaceutically acceptable carrier or other medicinal agents, pharmaceutical agents, carriers, adjuvants, diluents, etc. For injection, the carrier will typically be a liquid carrier. For other methods of administration, the carrier may be either solid or liquid, such as sterile pyrogen-free water or sterile pyrogen-free phosphate-buffered saline solution. For inhalation administration, the carrier is respirable, and preferably is in a solid or liquid particulate form. As an injection medium, it is preferred to use water that contains the additives that are common for injection solutions, such as stabilizing agents, salts or saline, and/or buffers.

In other embodiments, the present invention relates to a pharmaceutical composition comprising a cell, in which the rAAV5 vector is integrated into the genome, in a pharmaceutically acceptable carrier or other medicinal agents, pharmaceutical agents, carriers, adjuvants, diluents, etc.

"Pharmaceutical composition" means a composition comprising the above rAAV5 vector of the invention and at least one of components selected from the group consisting of pharmaceutically acceptable and pharmacologically compatible excipients, such as fillers, solvents, diluents, carriers, auxiliary, distributing agents, delivery agents, preservatives, stabilizers, emulsifiers, suspending agents, thickeners, prolonged delivery controllers, the choice and proportions of which depend on the type and route of administration and dosage. The pharmaceutical compositions of the present invention and methods of preparation thereof will be undoubtedly apparent to those skilled in the art. The pharmaceutical compositions should preferably be manufactured in compliance with the GMP (Good Manufacturing Practice) requirements. The composition may comprise a buffer composition, tonicity agents, stabilizers and solubilizers.

"Pharmaceutically acceptable" means a material that does not have biological or other negative side effects, for example, the material can be administered to a subject without causing any undesirable biological effects. Thus, such pharmaceutical compositions may be used, for example, in transfection of a cell ex vivo or in administration in vivo of a viral particle or a cell directly to a subject.

The term "excipient" is used herein to describe any ingredient other than the above ingredients of the invention. These are substances of inorganic or organic nature which are used in the pharmaceutical production/manufacturing in order to give drug products the necessary physicochemical properties.

"Stabilizer" refers to an excipient or a mixture of two or more excipients that provide the physical and/or chemical stability of the active agent.

The term "buffer", "buffer composition", "buffering agent" refers to a solution, which is capable of resisting changes in pH by the action of its acid-base conjugate components, which allows the rAAV5 vector product to resist changes in pH. Generally, the pharmaceutical composition preferably has a pH in the range from 4.0 to 8.0. Examples of buffers used include, but are not limited to, acetate, phosphate, citrate, histidine, succinate, etc. buffer solutions.

The pharmaceutical composition is "stable" if the active agent retains physical stability and/or chemical stability and/or biological activity thereof during the specified shelf life at storage temperature, for example, of 2-8 °C. Preferably, the active agent retains both physical and chemical stability, as well as biological activity. Storage period is adjusted based on the results of stability test in accelerated or natural aging conditions.

A pharmaceutical composition according to the invention may be manufactured, packaged, or widely sold in the form of a single unit dose or a plurality of single unit doses in the form of a ready formulation. The term "single unit dose" as used herein refers to discrete quantity of a pharmaceutical composition containing a predetermined quantity of an active ingredient. The quantity of the active ingredient typically equals the dose of the active ingredient to be administered in a subject, or a convenient portion of such dose, for example, half or a third of such dose.

### Method for delivering gene product

In one aspect, the present invention relates to a method for delivering a gene product to a subject in need thereof, comprising administering to the subject any of the above rAAV5 vectors or the above pharmaceutical composition.

Subject refers to any living organism that is amenable to the techniques provided in the present description. In certain non-limiting embodiments, the subject is a human. Said subject may be either male or female, of any age.

Any method for administering an rAAV5 vector, which is recognized in the art, can be suitably used for the above rAAV5 vector of the present invention.

Exemplary modes of administration include topical application, intranasal, inhalation, transmucosal, transdermal, enteral (e.g. oral, rectal), parenteral (e.g. intravenous, subcutaneous, intradermal, intramuscular) administrations, as well as direct tissue or organ injections.

Injectables may be prepared in conventional forms, either as liquid solutions or suspensions, solid forms suitable for the preparation of solution or suspensions in liquid prior to injection, or as emulsions. Alternatively, one may administer the above AAV5-based recombinant virus of the present invention in a local rather than systemic manner, for example in a depot or sustained-release formulation.

The AAV5-based recombinant virus is introduced into an organism in an effective amount. The AAV5-based recombinant virus is preferably introduced into an organism in a biologically effective amount. A "biologically effective" amount of the recombinant virus is an amount that is sufficient to cause infection (or transduction) and expression of the nucleic acid sequence in the cell. If the virus is administered to a cell in vivo (e.g. the virus is administered to a subject, as described below), a "biologically-effective" amount of the viral vector is an amount that is sufficient to cause the transduction and expression of the nucleic acid sequence in the target cell.

The cell for administering the above AAV5-based recombinant virus of the invention may be a cell of any type, including but not limited to motor neurons or other tissues of the nervous system, epithelial cells (e.g. skin, respiratory and gut epithelial cells), hepatic cells, muscle cells, pancreatic cells (including islet cells), hepatic cells, spleen cells, fibroblasts, endothelial cells, and the like.

The above AAV5-based recombinant virus of the invention is not used to modify the genetic integrity of human germ line cells.

### Use

In one aspect, the present invention relates to the use of any of the above rAAV5 vectors or the above pharmaceutical composition for treating a disease in a subject in need thereof.

In some embodiments of the use, the disease is selected from the group: blood diseases; central nervous system diseases; metabolic diseases; muscle diseases; hereditary diseases.

Subject refers to any animal that is amenable to the techniques provided in the present description. In certain non-limiting embodiments, the subject is a human. Said subject may be either male or female, of any age.

Administration of the rAAV5 vector of the present invention to a human subject or an animal in need thereof can be by any means known in the art for administering viral vectors.

Exemplary modes of administration include topical application, intranasal, inhalation, transmucosal, transdermal, enteral (e.g. oral, rectal), parenteral (e.g. intravenous, subcutaneous, intradermal, intramuscular) administrations, as well as direct tissue or organ injections.

Injectables may be prepared in conventional forms, either as liquid solutions or suspensions, solid forms suitable for the preparation of solution or suspensions in liquid prior to injection, or as emulsions. Alternatively, one may administer the above AAV5-based recombinant virus of the present invention in a local rather than systemic manner, for example in a depot or sustained-release formulation.

In some embodiments of the use, the disease is selected from the group comprising: blood diseases; central nervous system diseases; metabolic diseases; muscle diseases; hereditary diseases.

In some embodiments of the use, the disease is a blood disease.

In some embodiments of the use, the disease is a muscle disease.

In some embodiments of the use, the disease is a hereditary disease.

In some embodiments of the use, the expression product of the heterologous nucleic acid sequence is Factor IX or a functional variant thereof.

In some embodiments of the use, the expression product of the heterologous nucleic acid sequence is Factor VIII or a functional variant thereof.

In some embodiments of the use, the product of expression of the heterologous nucleic acid sequence is an SMN1 protein (survival motor neuron protein)

In some embodiments of the use, the product of expression of the heterologous nucleic acid sequence is an RBD-S polypeptide (recombinant receptor-binding domain of S glycoprotein) of SARS-cov2 (severe acute respiratory syndrome coronavirus 2).

In some embodiments of the use, any of the above rAAV5 vectors or the above pharmaceutical composition are used in therapeutically effective amounts.

A "therapeutically-effective amount" is understood to mean an amount that is sufficient to alleviate (e.g. mitigate, decrease, reduce) at least one of the symptoms associated with a disease state. Alternatively stated, a "therapeutically-effective" amount is an amount that is sufficient to provide some improvement in the condition of the subject.

Dosages of the above AAV5-based recombinant virus of the invention will depend on the mode of administration, the particular viral vector, and they can be determined in a routine manner. Exemplary doses for achieving therapeutic effects are viral titers of at least about 10⁵, 10⁶, 10⁷, 10⁸, 10⁹, 10¹⁰, 10¹¹, 10¹², 10¹³, 10¹⁴, 10¹⁵, 10¹⁶ transducing units or more, preferably about 10⁹ to 10¹⁵ transducing units, yet more preferably 10¹⁴ transducing units per kilogram.

Thus, the AAV5-based recombinant virus, reagents, and methods of the present invention can be used to direct a nucleic acid to either dividing or non-dividing cells, and to stably express the heterologous nucleic acid therein. Using this vector system, it is now possible to introduce into cells under in vivo conditions the genes that encode proteins that affect cell physiology. The vectors of the present invention can thus be useful in gene therapy for disease states.

In general, the present invention may be employed to deliver any foreign nucleic acid with a biological effect to treat or ameliorate the symptoms associated with any disorder related to gene expression. Exemplary disease states include, but are not limited to: cystic fibrosis (and other lung diseases), hemophilia A, hemophilia B, thalassemia, anemia and other blood coagulation disorders, AIDs, Alzheimer's disease, Parkinson's disease, Huntington's disease, amyotrophic lateral sclerosis, epilepsy, and other neurological disorders, diabetes mellitus, muscular dystrophies (e.g. Duchenne, Becker, spinal muscular atrophy (SMA)), Gaucher's disease, Hurler's disease, adenosine deaminase deficiency, glycogen storage diseases and other metabolic defects, diseases of solid organs (e.g. brain, liver, kidney, heart), and the like.

Gene transfer has substantial potential use in understanding and providing therapy for disease states. There are a number of hereditary diseases for which defective genes are known and have been cloned. In some cases, the function of these cloned genes is known. In general, the above disease states fall into two classes: deficiency states, typically enzyme deficiency, which are generally inherited in a recessive manner, and unbalanced states, sometimes involving at least regulatory or structural proteins, which are inherited in a dominant manner. For deficiency state diseases, gene transfer could be used to bring a normal gene into affected tissues for replacement therapy. For unbalanced disease states, gene transfer could be used to create a disease state in a model system, which could then be used in efforts to counteract the disease state. Thus, the methods of the present invention permit to treat genetic diseases. According to the invention, a disease state is treated by partially or wholly remedying the deficiency or imbalance that causes the disease or makes it more severe. The use of site-specific integration of nucleic sequences to induce mutations or to correct deficiencies is also possible.

### Method for producing rAAV5 vector

In one aspect, the present invention relates to a method for producing any of the above rAAV5 vectors, comprising transfecting producer cells with any of the above nucleic acids comprising a modified capsid protein VP1 from adeno-associated virus serotype 5 (AAV5) or with the above nucleic acid encoding a capsid.

Capsid proteins may be expressed from a recombinant virus, an expression vector, or from a cell line into which the genes of the subject modified AAV capsids or coding sequences are stably integrated. Furthermore, the invention provides the production of AAV capsids with the described mutations in vitro from AAV capsid proteins and the construction of packaged capsids in vitro. The invention further provides the production of modified AAV capsids that have been genetically engineered to express heterologous epitopes of clinically significant antigens in order to elicit an immune response.

The method for producing the rAAV5 vector has been described in detail in the examples section.

### Transgenic animal

According to some aspects of the disclosure, provided is a method for creating a somatic transgenic animal model. In some embodiments, the method comprises administering any of the above rAAV to a non-human animal, wherein the rAAV comprises at least one transgene, and wherein the rAAV infects cells of the target tissue of a non-human animal.

In some embodiments, the use is possible of the subject capsid variants to create a somatic transgenic animal model, comprising administering any of the above rAAV to a non-human animal, wherein the rAAV comprises at least one transgene. In some embodiments, the transgene is at least one gene encoding a protein. In some embodiments, the transgene encodes at least one small inhibitory nucleic acid. In some embodiments, the transgene encodes at least one reporter molecule.

The somatic transgenic animal model may be a mammal, such as a mouse, a rat, a rabbit, a dog, a cat, a sheep, a pig, a non-human primate.

In some embodiments a putative therapeutic agent may be administered to the somatic transgenic animal model to determine the effect of the putative therapeutic agent on the disease state in the animal.

### Examples

The following examples are provided for better understanding of the invention. These examples are for purposes of illustration only and are not to be construed as limiting the scope of the invention in any manner.

All publications, patents, and patent applications cited in this specification are incorporated herein by reference. Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended embodiments.

### Materials and general methods

### Recombinant DNA techniques

Standard methods were used to manipulate DNA as described in Sambrook, J. et al, Molecular cloning: A laboratory manual; Cold Spring Harbor Laboratory Press, Cold Spring Harbor, New York, 2012. The molecular biological reagents were used according to the manufacturer protocols.

### Gene synthesis

Desired gene segments were prepared from oligonucleotides made by chemical synthesis. The gene segments of 300-4,000 bp long, which were flanked by singular restriction sites, were assembled by annealing and ligation of oligonucleotides including PCR amplification and subsequently cloned via the specified restriction sites. The DNA sequences of the subcloned gene fragments were confirmed by DNA sequencing.

### DNA sequence determination

DNA sequences were determined by Sanger sequencing.

### DNA and protein sequence analysis and sequence data management

SnapGene software package version 5.1.4.1 was used for sequence creation, mapping, analysis, annotation and illustration.

### Statistical data analysis

The results indicate an average value ± standard deviation (SD), analysis of variance (ANOVA) was employed to compare the test and control results, and they were determined to be statistically significant.

### Example 1. Production of libraries of rAAV5 capsid variants

Libraries of rAAV5 capsid variants were produced by random mutagenesis of the Cap gene sequence (Davidsson M. et al., 2016). Briefly, the Cap gene sequence of serotype 5 comprising the mutations S2A and T711S in VP1 which were produced in the previous round of selection (patent application No. RU2019126509) was fragmented using uracil-DNA glycosylase, the resulting fragments were assembled into a full-length Cap gene using DNA polymerase not having proofreading activity (resulting in random mutations in the sequence). The full-length mutant variants were cloned into the carrier plasmid pAAV-linker (Fig. 1) at AscI/EcoRI restriction sites in a common reading frame with green fluorescent protein (GFP), thereby producing a diverse random library of rAAV5 capsids, which library was thereafter used to select capsid variants having increased transduction activity.

Viral particles having increased transducing activity were positively selected in vitro on CHO-K1-S cells. Further, for transduction, we used particles that were purified using ultracentrifugation in a iodixanol gradient. After 48 hours, the cells were harvested and genomic DNA was isolated for subsequent amplification of the viral genome sequences capable of efficient transduction. The resulting sequences were thereafter re-cloned and re-produced for subsequent selection iterations to enrich the library with variants having the highest transduction efficiency. After 5 rounds of selection, the capsid genes of 100 clones were sequenced to determine the most successful mutations and combinations thereof. According to the sequencing results, the predominant combinations of mutations were S2A, T711S, T614V in AAV5 VP1 amounting to about 30% of the clones. Capsid variants comprising the mutations S2A, T711S, T614A in AAV5 VP1 and S2A, T711S, G226V in AAV5 VP1 were also selected. These capsid variants were cloned into vectors for producing viral particles and further used for visualizing and comparing the transduction profiles relative to wild-type AAV5.

### Example 2. Generation followed by selection of recombinant viral particles from resulting sequence library

To produce and subsequently select recombinant viral particles from the resulting sequence library, a series of plasmids was developed as follows: a carrier plasmid, a plasmid comprising the Rep gene sequence, as well as a construct comprising adenoviral genes that are required for the replication of viral particles.

The carrier plasmid pAAV-linker (Fig. 1), intended for cloning the libraries of random variants of the capsid gene of AAV serotype 5 into one reading frame with the reporter protein, was produced by substituting the sequence of a modified green fluorescent protein in the original construct pAAV-GFP (Fig. 2), using the restrictase-ligase method of cloning at HindIII/EcoRI sites, for the sequence T2A-GFP synthesized *de novo* with the addition of an EcoRI restriction site from the 5' end and a HindIII restriction site from the 3' end.

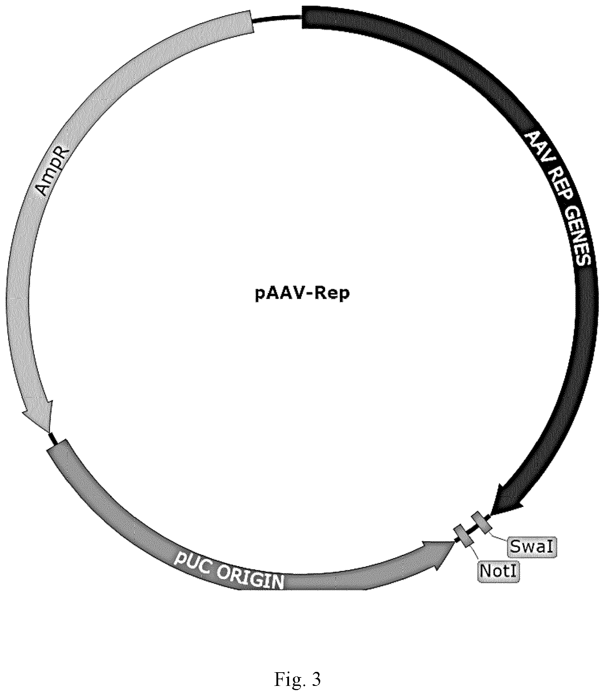

The plasmid pAAV-Rep comprising the Rep gene sequence (Fig. 3 was produced by de novo cloning of the synthesized sequence of the AAV serotype 2 Rep gene (GenBank ID AF043303.1) at PciI/PsiI restriction sites with subsequent treatment with T4 DNA Polymerase to generate blunt ends into the plasmid pGem-T Easy (Promega, USA) also treated with PciI/PsiI restriction enzymes.

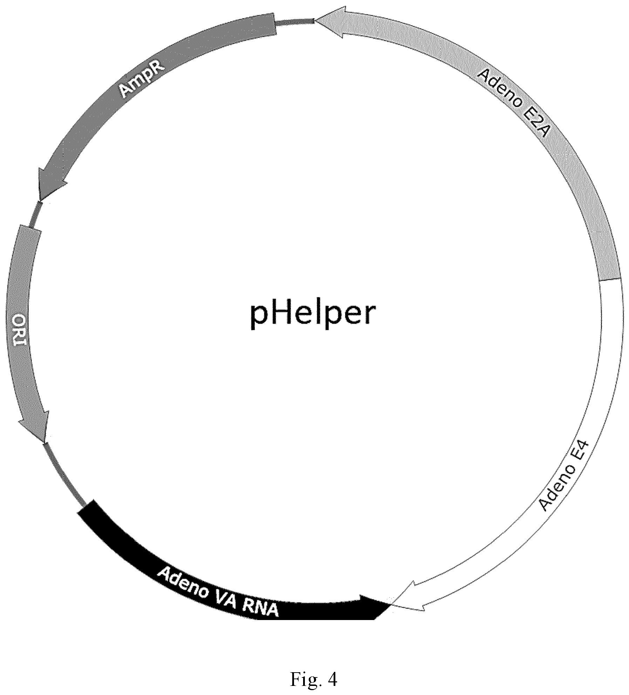

The adenoviral genes for producing the recombinant viral particles were sourced from the construct pHelper (Fig. 4), comprising AmpR - a beta-lactamase gene that provides resistance to ampicillin, Ori - a replication origin in bacteria, Adeno E2A - a helper adenovirus gene sequence involved in viral DNA replication, Adeno E4 - a helper adenovirus gene sequence involved in viral DNA replication, Adeno VARNA - a helper adenovirus gene sequence responsible for the translation of both early and late viral genes.

### Example 3. Method for producing vectors based on modified adeno-associated virus serotype 5 (rAAV5)

To produce rAAV particles with a modified serotype 5 capsid, producer cells were transfected simultaneously with 3 plasmids as follows:
1) With a plasmid comprising adenovirus nucleotide sequences encoding proteins and RNAs required for assembly of rAAV particles (helper plasmid);
2) With a plasmid comprising the natural nucleotide sequence of the Rep gene of adeno-associated virus, as well as the sequence of modified Cap gene, which is selected from the group: the nucleotide sequence of SEQ ID NO: 10, 11, 12, 13, 14, 15, 16 or any other nucleotide sequence encoding the VP1 protein with the amino acid sequence of SEQ ID No: 2, 3, 4, 5, 6, 7 or 8, and the VP2 and VP3 proteins from alternative reading frames of the nucleotide sequence being used, wherein
   VP2 may have any of the amino acid sequences of SEQ ID No: 18, 19, 20, 21, 22, 23 or 24;
   and VP3 may have any of the amino acid sequences of SEQ ID No: 34, 35, 36, 37, 38, 39 or 40;
3) With a plasmid comprising a heterologous rAAV particle genome encoding the target gene intended for delivery into patient's cells.

This set of genes provides the assembly of rAAV viral particles and encapsidation of the target genome therein within 72 hours. 72 hours following transfection, the producer cells are lysed to release rAAV particles for purification by subsequent filtration and chromatography steps. The titer of the purified rAAV particles is verified by enzyme linked immunosorbent assay and quantitative PCR.

### Example 4. Increasing the efficiency of cell transduction with rAAV5-based products in the presence of mutations S2A, T614V/T614A/G226V, T711S in wild-type AAV5 capsid protein VP1.

### Experimental design:

The CHO-K1-S cells were plated into the wells of 12-well plates. Seeding was made into the following growth medium: DMEM/F12 supplemented with glutamine, glucose content was 4.5 g/l, 5% bovine serum. Cell seeding density was 10,000 cell/cm². During the transduction run, pre-prepared cells were transduced at MOI of 500, 1,250 and 2,500 vg/cell. All samples were run in triplicates. Intact cells were used as a negative control.

rAAV5-based products with the mutations S2A and T711S in the capsid protein VP1, and a rAAV5-based product with a wild-type capsid were used as controls. It was previously shown (patent application No. RU2019126509) that the presence of the mutations S2A and T711S in the AAV5 capsid protein VP1 causes significantly higher efficiency of CHO-K1-S cell transduction compared to the wild-type capsid.

Analysis of transduction efficiency was performed using the Guava EasyCyteflow cytometer and the GuavaSoft software.

The authors of the invention surprisingly found that the presence of one or more mutations selected from the group: T614V, T614A and G226V in the wild-type rAAV5 capsid protein VP1 or in the rAAV5 capsid protein VP1 already comprising the mutations S2A and T711S caused a significant increase in the efficiency of transgene delivery by rAAV vectors with said mutations (T614V, T614A and G226V). For example, flow cytometry allowed to detect a change in the number of GFP positive cells 48 hours following the transduction of CHO-K1-S line with rAAV5 products containing the wild-type protein VP1, containing protein VP1 comprising the mutations S2A and T711S, or protein VP1 carrying the mutations S2A, T711S and T614V or T614A or G226V (Fig. 5).

In the presence of the mutations T614V, S2A and T711S (AAV5-02Mut-GFP), the number of cells expressing GFP increased by 1.31 times from 24.92% to 32.84% at MOI 500, by 1.47 times from 43.44% to 63.89% at MOI 1,250 and by 1.17 times from 65.56% to 78.01% at MOI 2,500 vg/cell compared to the control rAAV5 product with the capsid protein VP1 comprising only the mutations S2A and T711S (AAV5-01Mut-GFP). When comparing the rAAV5 product with the capsid protein VP1 comprising the mutations T614V, S2A and T711S (AAV5-02Mut-GFP) and wild-type capsid VP1 (AAV5-NullMut-GFP), the number of cells expressing GFP increased by 2.74 times from 11.97% to 32.84% at MOI 500, by 3.18 times from 20.11% to 63.89% at MOI 1,250 and 1.85 times from 42.26% to 78.01% at MOI 2,500

In the presence of the mutations T614A, S2A and T711S (AAV5-03Mut-GFP), the number of cells expressing GFP increased by 1.18 times from 24.92% to 29.62% at MOI 500, 1.27 times from 43.44% to 55.22% at MOI 1,250 and 1.1 times from 65.56% to 72.05% at MOI 2,500 vg/cell compared to the control rAAV5 product with the capsid protein VP1 comprising only the mutations S2A and T711S (AAV5-01Mut-GFP). When comparing the rAAV5 product with the capsid protein VP1 comprising the mutations T614A, S2A and T711S (AAV5-03Mut-GFP) and wild-type capsid VP1 (AAV5-NullMut-GFP), the number of cells expressing GFP increased by 2.47 times from 11.97% to 29.62% at MOI 500, 2.74 times from 20.11% to 55.22% at MOI 1,250 and 1.7 times from 42.26% to 72.05% at MOI 2,500.

In the presence of the mutations G226V, S2A and T711S (AAV5-04Mut-GFP), the number of cells expressing GFP decreased by 1.41 times from 24.92% to 17.67% at MOI 500, 1.09 times from 43.44% to 39.69% at MOI 1,250 and 1.05 times from 65.56% to 61.85% at MOI 2,500 vg/cell compared to the control rAAV5 product with the capsid protein VP1 comprising only the mutations S2A and T711S (AAV5-01Mut-GFP). When comparing the rAAV5 product with the capsid protein VP1 comprising the mutations T614A, S2A and T711S (AAV5-03Mut-GFP) and wild-type capsid VP1 (AAV5-NullMut-GFP), the number of cells expressing GFP increased by 1.48 times from 11.97% to 17.67% at MOI 500, 1.97 times from 20.11% to 39.69% at MOI 1,250 and 1.46 times from 42.26% to 61.85% at MOI 2,500.

### Example 5. Efficiency of AAV viral genome packaging with rAAV5-based products in the presence of mutations S2A, T614V/G226V, T711S in wild-type AAV5 capsid protein VP1.

As used in the present invention, the terms "vector genome" and "vector DNA" are intended to mean ssDNA packaged in the capsid of recombinant adeno-associated virus (hereinafter referred to as rAAV). Such ssDNA can be both a sense one (hereinafter referred to as plus DNA strand) and an antisense one (hereinafter referred to as minus DNA strand). The rAAV product is a mixture of capsids, some of which comprise the plus ssDNA strand, and some comprise the minus ssDNA strand. The rAAV vector genome is a nucleotide sequence of an expression cassette surrounded by a sequence of inverted terminal repeats, or ITRs (hereinafter referred to as ITRs).

As used in the present invention, the terms "sample" or "test sample" are intended to mean ssDNA extracted from the corresponding rAAV product.

According to the literature, AAV capsids are capable of efficient packaging of cassettes up to 4.8 kb, whereas larger cassettes while being packaged into a capsid are fragmented from the 5' or 3' ends of DNA, resulting in a less efficient packaging and thus forming a heterogeneous population of rAAV viral genomes. Further, the 5' end of single-stranded DNA (hereinafter referred to as ssDNA) is most often deleted (Zhijian Wu, 2010.). Thus, a significant part of rAAV products contain fragmented ssDNA strands, leading to decreased efficiency of expression. Southern dot-blotting was used to measure the efficiency of ssDNA packaging with rAAV capsids. To check the integrity of 5' and 3' ends of ssDNA in the test sample, oligonucleotide probes were selected for DNA regions that are adjacent to the 5' ITR and 3' ITR sequences. The probes should bind to the selected ssDNA regions with strict specificity. Further, we selected the probes for DNA regions corresponding to the middle of the expression cassette (~2400-2500 bp); this detection point is a control point for the test ssDNA strand, since this region is less likely to be subjected to fragmentation when packaged into a capsid (Fig. 6.). The oligonucleotide probes were selected for both the "plus" and "minus" DNA strands of the expression cassette. Probes differ from conventional oligonucleotides in that there is a biotin molecule at the 5' end of the sequence.

In the course of the study, we applied the test sample, i.e. DNA extracted from the tes rAAV products, onto a nitrocellulose membrane pre-soaked in a 20XSS buffer, and hybridized with selected probes. A separate membrane with test samples was used for each probe; 6 membranes were thus processed, each of which contained test DNA samples extracted from 2.5×10¹⁰ viral particles (measured as GC - genome copy) with pretreatment of the viral product with DNase I to remove impurities of non-encapsidated DNA followed by treatment with Proteinase K, according to the protocol described in the article (Serotype-dependent packaging of large genes in adeno-associated viral vectors results in effective gene delivery in mice Mariacarmela Allocca, J Clin Invest. 2008 May 1; 118(5): 1955-1964.)

The samples were applied onto the membrane using the Bio-Dot^{®} Microfiltration Apparatus (Bio-Rad) vacuum manifold. The extracted DNA from 2.3 × 10⁹ vector genomes was applied to the first well of the manifold. Twofold serial dilutions of the sample were applied to the remaining six wells of the manifold to produce thereby a series of dilutions corresponding to amounts 7, 3.5, 1.7, 0.86, 0.43, 0.21 and 0.11 ng of vector DNA. Standard pDNA produced by cleaving pAAV-GOI at SmaI sites was diluted in the same fashion. Amount of standard pDNA in the calibration curve was 50, 25, 12.5, 6.25, 3.1, 1.6, 0.78, respectively.

As used in the present invention, the term "Standard pDNA" is intended to mean plasmid DNA cleaved at SmaI sites (hereinafter referred to as pDNA), the nucleotide sequence of which exactly corresponds to the nucleotide sequence of the vector genome under study.

The probes used for hybridization are synthesized and labeled with biotin. Hybridization was carried out overnight at a temperature 10 °C below the melting point of each probe.

The next day, the membranes were washed first with a solution of 2 × SSC/0.1% SDS, and then with TBSTx1. The membranes were then incubated with 1% BSA and TBSTx1 for 40 minutes and TBSTx1 was washed again. Next, the membranes were incubated with HRP-conjugated streptavidin for an hour to bind the biotinylated probe to HRP-conjugated streptavidin. Following the final washing of the membranes with 1% BSA TBSTx1, a chemiluminescent detection substrate was added and a luminescent signal was detected on the membranes (Fig. 7). The resulting images were further processed using the "Image Lab" software. The intensity of probe luminescent signals was measured by the level of intensity of the standard pDNA luminescent signal. The intensity of the luminescent signal of standard pDNA dilutions was taken as a calibration curve (hereinafter referred to as standard sample).

The authors of the invention found that the selected biotinylated probes bind to the DNA nanomatrix present on the membrane with the same efficiency, which is evident from the staining of standard pDNA dilutions at each point. DNA fragments corresponding to the 5' and 3' ends of the plus strand and the 5' and 3' ends of the minus strand of DNA under study were detected in all test samples. With the same load of test sample DNAs on the membranes, differences in the intensity of the detected luminescence signals were observed.

In the detection region of "Probe 1", the intensity of the luminescence signal at the first dilution point of the sample vgDNA-04Mut-GFP corresponds to that of the third dilution of the standard sample pDNA-GFP. The limit of detection for the sample vgDNA-04Mut-GFP is detected at the sixth dilution, the seventh dilution of the sample is detected at the level of background signal.

In the detection region of "Probe 1", the intensity of the luminescence signal at the first dilution point of the sample vgDNA-02Mut-GFP is in the range of intensity of the luminescence signals of the third and fourth dilutions of the standard sample pDNA-GFP. The limit of detection for the sample vgDNA-02Mut-GFP is detected at the fifth dilution, the sixth and seventh dilutions of the sample are detected at the level of background signal.

In the detection region of "Probe 1", the intensity of the luminescence signal at the first dilution point of the sample vgDNA-NullMut-GFP corresponds to that of the fourth dilution of the standard sample pDNA-GFP. The limit of detection for the sample vgDNA-NullMut-GFP is detected at the fourth dilution; the fifth, sixth and seventh dilutions of the sample are detected at the level of background signal.

When comparing the intensity of the luminescence signals of the samples in the "Probe 1" detection region, the samples vgDNA-04Mut-GFP and vgDNA-02Mut-GFP have a greater luminescence signal strength compared to the control sample vgDNA-NullMut-GFP. And is detected at further dilutions relative to the control sample vgDNA-NullMut-GFP.

In the detection region of "Probe 3", the intensity of the luminescence signal at the first dilution point of the sample vgDNA-04Mut-GFP is beyond the dilution of the standard sample pDNA-GFP. The intensity of the luminescence signal at the second dilution point of the sample vgDNA-04Mut-GFP is in the range of the intensity of the luminescence signals of the first and second dilutions of the standard sample pDNA-GFP. The limit of detection for the sample vgDNA-04Mut-GFP is detected at the fifth dilution, the sixth and seventh dilutions of the sample are detected at the level of background signal.

In the detection region of "Probe 3", the intensity of the luminescence signal at the first dilution point of the sample vgDNA-02Mut-GFP is beyond the dilution of the standard sample pDNA-GFP. The intensity of the luminescence signal at the second dilution point of the sample vgDNA-02Mut-GFP is in the range of the intensity of the luminescence signals of the first and second dilutions of the standard sample pDNA-GFP. The limit of detection for the sample vgDNA-02Mut-GFP is detected at the fifth dilution, the sixth and seventh dilutions of the sample are detected at the level of background signal.

In the detection region of "Probe 3", the intensity of the luminescence signal at the first dilution point of the sample vgDNA-NullMut-GFP is beyond the dilution of the standard sample pDNA-GFP. The intensity of the luminescence signal at the second dilution point of the sample vgDNA-NullMut-GFP is in the range of the intensity of the luminescence signals of the second and third dilutions of the standard sample pDNA-GFP. The limit of detection for the sample vgDNA-NullMut-GFP is detected at the fourth dilution; the fifth, sixth and seventh dilutions of the sample are detected at the level of background signal.

When comparing the intensity of the luminescence signals of the samples in the "Probe 3" detection region, the samples vgDNA-04Mut-GFP and vgDNA-02Mut-GFP have a greater luminescence signal strength compared to the control sample vgDNA-NullMut-GFP. And is detected at further dilutions relative to the control sample vgDNA-NullMut-GFP.

In the detection region of "Probe 5", the intensity of the luminescence signal at the first dilution point of the sample vgDNA-04Mut-GFP corresponds to that of the fifth dilution of the standard sample pDNA-GFP. The limit of detection for the sample vgDNA-04Mut-GFP is detected at the fourth dilution; the fifth, sixth and seventh dilutions of the sample are detected at the level of background signal.

In the detection region of "Probe 5", the intensity of the luminescence signal at the first dilution point of the sample vgDNA-02Mut-GFP corresponds to that of the fourth dilution of the standard sample pDNA-GFP. The limit of detection for the sample vgDNA-02Mut-GFP is detected at the seventh dilution.

In the detection region of "Probe 5", the intensity of the luminescence signal at the first dilution point of the sample vgDNA-NullMut-GFP corresponds to that of the fifth dilution of the standard sample pDNA-GFP. The limit of detection for the sample vgDNA-NullMut-GFP is detected at the fifth dilution; the sixth and seventh dilutions of the sample are detected at the level of background signal.

When comparing the intensity of the luminescence signals of the samples in the "Probe 5" detection region, the sample vgDNA-04Mut-GFP does not differ from the control sample vgDNA-NullMut-GFP. The sample vgDNA-02Mut-GFP has a higher luminescence signal strength compared to the control sample vgDNA-NullMut-GFP. And is detected at further dilutions relative to the control sample vgDNA-NullMut-GFP.

In the detection region of "Probe 2", the intensity of the luminescence signal at the first dilution point of the sample vgDNA-04Mut-GFP is in the range of intensity of the luminescence signals of the fourth and fifth dilutions of the standard sample pDNA-GFP. The limit of detection for the sample vgDNA-04Mut-GFP is detected at the fourth dilution; the fifth, sixth and seventh dilutions of the sample are detected at the level of background signal.

In the detection region of "Probe 2", the intensity of the luminescence signal at the first dilution point of the sample vgDNA-02Mut-GFP is in the range of intensity of the luminescence signals of the fourth and fifth dilutions of the standard sample pDNA-GFP. The limit of detection for the sample vgDNA-02Mut-GFP is detected at the fifth dilution; the sixth and seventh dilutions of the sample are detected at the level of background signal.

In the detection region of "Probe 2", the intensity of the luminescence signal at the first dilution point of the control sample vgDNA-NullMut-GFP is in the range of intensity of the luminescence signals of the fourth and fifth dilutions of the standard sample pDNA-GFP. The limit of detection for the sample vgDNA-NullMut-GFP is detected at the fourth dilution; the fifth, sixth and seventh dilutions of the sample are detected at the level of background signal.

When comparing the intensity of the luminescence signals of the samples in the "Probe 2" detection region, the sample vgDNA-04Mut-GFP does not differ from the control sample vgDNA-NullMut-GFP. The sample vgDNA-02Mut-GFP has a higher luminescence signal strength compared to the control sample vgDNA-NullMut-GFP. And is detected at further dilutions relative to the control sample vgDNA-NullMut-GFP.

In the detection region of "Probe 4", the intensity of the luminescence signal at the first dilution point of the sample vgDNA-04Mut-GFP is beyond the dilution of the standard sample pDNA-GFP. The intensity of the luminescence signal at the second dilution point of the sample vgDNA-04Mut-GFP is in the range of the intensity of the luminescence signals of the first and second dilutions of the standard sample pDNA-GFP. The limit of detection for the sample vgDNA-04Mut-GFP is detected at the sixth dilution, the seventh dilution of the sample is detected at the level of background signal.

In the detection region of "Probe 4", the intensity of the luminescence signal at the first dilution point of the sample vgDNA-02Mut-GFP is in the range of intensity of the luminescence signals of the first and second dilutions of the standard sample pDNA-GFP. The limit of detection for the sample vgDNA-02Mut-GFP is detected at the sixth dilution, the seventh dilution of the sample is detected at the level of background signal.

In the detection region of "Probe 4", the intensity of the luminescence signal at the first dilution point of the sample vgDNA-NullMut-GFP is in the range of intensity of the luminescence signals of the first and second dilutions of the standard sample pDNA-GFP. The limit of detection for the sample vgDNA-NullMut-GFP is detected at the fifth dilution; the sixth and seventh dilutions of the sample are detected at the level of background signal.

When comparing the intensity of the luminescence signals of the samples in the "Probe 4" detection region, the samples vgDNA-04Mut-GFP and vgDNA-02Mut-GFP have a greater luminescence signal strength compared to the control sample vgDNA-NullMut-GFP. And is detected at further dilutions relative to the control sample vgDNA-NullMut-GFP.

In the detection region of "Probe 6", the intensity of the luminescence signal at the first dilution point of the sample vgDNA-04Mut-GFP is in the range of intensity of the luminescence signals of the fourth and fifth dilutions of the standard sample pDNA-GFP. The limit of detection for the sample vgDNA-04Mut-GFP is detected at the sixth dilution, the sixth and seventh dilutions of the sample are detected at the level of background signal.

In the detection region of "Probe 6", the intensity of the luminescence signal at the first dilution point of the sample vgDNA-02Mut-GFP corresponds to that of the fourth dilution of the standard sample pDNA-GFP. The limit of detection for the sample vgDNA-02Mut-GFP is detected at the fourth dilution; the fifth, sixth and seventh dilutions of the sample are detected at the level of background signal.

In the detection region of "Probe 6", the intensity of the luminescence signal at the first dilution point of the sample vgDNA-NullMut-GFP corresponds to that of the fourth dilution of the standard sample pDNA-GFP. The limit of detection for the sample vgDNA-NullMut-GFP is detected at the fourth dilution; the fifth, sixth and seventh dilutions of the sample are detected at the level of background signal.

When comparing the intensity of the samples' luminescence signals in the "Probe 6" detection region, the sample vgDNA-02Mut-GFP does not differ from the control sample vgDNA-NullMut-GFP, and the sample vgDNA-04Mut-GFP has a greater luminescence signal strength and is detected at later dilutions compared to the control sample vgDNA-NullMut-GFP.

When comparing the intensity of the luminescence signals of the sample vgDNA-04Mut-GFP at the detection points of "Probe 1" (3' region of minus DNA strand) and "Probe5" (5' region of minus DNA strand). The 3' region of minus DNA srand (Probe 1) has the highest intensity of the luminescence signal corresponding to that of the third dilution of the standard sample pDNA-GFP, compared to the detectable 5' region of minus DNA strand (Probe 5) corresponding to the intensity of the luminescence signal of the fifth dilution of the standard sample pDNA-GFP.

When comparing the intensity of the luminescence signals of the sample vgDNA-02Mut-GFP at the detection points of "Probe 1" (3' region of minus DNA strand) and "Probe 5" (5' region of minus DNA strand). The 3' region of minus DNA srand has the highest intensity of the luminescence signal, which is in the range of that of the third and fourth dilutions of the standard sample pDNA-GFP, compared to the detectable 5' region of minus DNA strand corresponding to the intensity of the luminescence signal of the fourth dilution of the standard sample pDNA-GFP.

When comparing the intensity of the luminescence signals of the control sample vgDNA-NullMut-GFP at the detection points of "Probe 1" (3' region of minus DNA strand) and "Probe 5" (5' region of minus DNA strand). The 3' region of minus DNA srand has the highest intensity of the luminescence signal corresponding to that of the fourth dilution of the standard sample pDNA-GFP, compared to the detectable 5' region of minus DNA strand corresponding to the intensity of the luminescence signal of the fifth dilution of the standard sample pDNA-GFP.

For the samples vgDNA-04Mut-GFP, vgDNA-02Mut-GFP, vgDNA-NullMut-GFP, the packaging efficiency for minus strands was measured based on differences in the intensity of the luminescence signal in the detection region of the 3' end of minus DNA srand and the intensity of the luminescence signal in the detection region of the 5' end of minus DNA strand. All the samples studied exhibited the tendency of the intensity of the luminescence signal in the detection region of the 3' end of minus DNA strand to prevail over that in the detection region of the 5' end of minus DNA strand. However, the comparison between the intensity of luminescence signals in the detection region of the 3' end of minus DNA strand and the that in the detection region of the 5' end of the minus DNA strand of the samples revealed that the samples vgDNA-04Mut-GFP and vgDNA-02Mut-GFP exhibited a more efficient packaging of minus DNA strand compared to the control sample vgDNA-NullMut-GFP because of a high luminescence signal strength both for the detection region of the 3' end of minus DNA strand and for the detection region of the 5' end of minus DNA strand; and is detected at later dilutions relative to the control sample vgDNA-NullMut-GFP. The sample vgDNA-02Mut-GFP exhibits more efficient packaging of minus DNA strand compared to vgDNA-04Mut-GFP and the control sample vgDNA-NullMut-GFP due to the fact that the intensity of luminescence signal in the detection region of the 3' end of minus DNA strand is in the range of that of the third and fourth dilutions of the standard sample pDNA-GFP, and the intensity of luminescence signal in the detection region of the 5' end of minus DNA strand corresponds to that of the fourth dilution of the standard sample pDNA-GFP, whereas the samples vgDNA-04Mut-GFP and vgDNA-NullMut-GFP exhibited the intensity of luminescence signal in the detection region of the 5' end of minus DNA strand which was significantly lower than that in the detection region of the 3' end of minus DNA strand of these samples. This fact indicates more efficient packaging of full-size minus DNA strands in the sample vgDNA-02Mut-GFP.

Comparison of the intensity of luminescence signals of the sample vgDNA-04Mut-GFP at the detection points of "Probe 6" (3' region of plus DNA strand) and "Probe 2" (5' region of plus DNA strand) showed no differences; the luminescence intensities at these points are in the range of the intensity of luminescence signal of the fourth and fifth dilutions of the standard sample pDNA-GFP.

Comparison of the intensity of luminescence signals of the sample vgDNA-02Mut-GFP at the detection points of "Probe 6" (3' region of plus DNA strand) and "Probe 2" (5' region of plus DNA strand) showed no differences; the luminescence intensities at these points are in the range of the intensity of luminescence signal of the fourth and fifth dilutions of the standard sample pDNA-GFP.

Comparison of the intensity of luminescence signals of the sample vgDNA-NullMut-GFP at the detection points of "Probe 6" (3' region of plus DNA strand) and "Probe 2" (5' region of plus DNA strand) showed no differences; the luminescence intensities at these points are in the range of the intensity of luminescence signal of the fourth and fifth dilutions of the standard sample pDNA-GFP.

For the samples vgDNA-04Mut-GFP, vgDNA-02Mut-GFP, vgDNA-NullMut-GFP, the packaging efficiency for plus strands was measured based on the intensity of luminescence signals in the detection region of the 3' end of plus DNA strand and in the detection region of the 5' end of plus DNA strand. It was found that all samples in the detection region of the 3' end of plus DNA strand showed the luminescence signal of test samples detectable in the range of the intensity of luminescence signals of the fourth and fifth dilutions of the standard sample pDNA-GFP, as well as the luminescence signal from detection of the 5' region of plus DNA strand of test samples was detected in the range of the intensity of luminescence signals of the fourth and fifth dilutions of the standard sample pDNA-GFP. Accordingly, the packaging of plus DNA strands in the samples under study has equal efficiency.

### Example 6. Efficiency of generation of vectors based on modified adeno-associated virus serotype 5 (rAAV5)

To produce rAAV particles with the modified serotype 5 capsid, HEK293 producer cells were transfected using polyethylenimine simultaneously with 3 plasmids as follows:
1) With a plasmid comprising adenovirus nucleotide sequences encoding proteins and RNAs required for assembly of rAAV particles (helper plasmid);
2) With a plasmid comprising the natural nucleotide sequence of the Rep gene of adeno-associated virus, as well as the sequence of modified Cap gene, which is selected from the group: the nucleotide sequence of SEQ ID NO: 10, 11, 12, 13, 14, 15, 16 or any other nucleotide sequence encoding the VP1 protein with the amino acid sequence of SEQ ID No: 2, 3, 4, 5, 6, 7 or 8, and the VP2 and VP3 proteins from alternative reading frames of the nucleotide sequence being used, wherein
   VP2 may have any of the amino acid sequences of SEQ ID No: 18, 19, 20, 21, 22, 23 or 24;
   and VP3 may have any of the amino acid sequences of SEQ ID No: 34, 35, 36, 37, 38, 39 or 40;
3) With a plasmid comprising a heterologous rAAV particle genome encoding the target gene intended for delivery into patient's cells.

This set of genes provides the assembly of rAAV viral particles and encapsidation of the target genome therein within 72 hours. 72 hours following transfection, the producing cells were subjected to lysis to release rAAV particles, the resulting vectors were treated with DNAase I for 2 hours at 37 °C, and then for another 2 hours with proteinase K at 56 °C. The titer of the resulting rAAV particles was determined by quantitative PCR using an oligonucleotide set consisting of a forward primer 5'- ACCACATGAAGCAGCACGAC -3', a reverse primer 5'-TCAGCTCGATGCGGTTCAC -3', and a probe 5'- HEX-CATGCCCGAAGGCTACGTCCAG-BHQ1 -3' specific for the GFP sequence.

The authors of the invention surprisingly found that the presence of one or more mutations selected from the group consisting of T614V, T614A or G226V in the wild-type rAAV5 capsid protein VP1 or in the rAAV5 capsid protein VP1 already comprising the mutations S2A and T711S caused a significant increase in the yield of encapsidated viral particles based on rAAV5 with said mutations (T614V, T614A and G226V) compared to wild-type rAAV5 capsid. For example, quantitative PCR showed a change in copy number of the packaged heterologous rAAV particle genome encoding the target GFP gene (Fig. 9).

In the presence of the mutations T614V, S2A and T711S (AAV5-02Mut-GFP), the number of packaged viral genomes increased by 7.09 times from 2.51E+09 vg/ml to 1.78E+10 vg/ml compared to the control rAAV5 product containing the wild-type capsid protein VP1 (AAV5-NullMut-GFP). When comparing the rAAV5 product containing the capsid protein VP1 comprising the mutations T614V, S2A and T711S (AAV5-02Mut-GFP) and capsid VP1 comprising only the mutations S2A and T711 S (AAV5-01Mut-GFP), the number of packaged viral genomes increased by 1.31 times from 1.36E+10 vg/ml to 1.78E+10 vg/ml

In the presence of the mutations T614A, S2A and T711S (AAV5-03Mut-GFP), the number of packaged viral genomes increased by 4.78 times from 2.51E+09 vg/ml to 1.20E+10 vg/ml compared to the control rAAV5 product containing the wild-type capsid protein VP1 (AAV5-NullMut-GFP). When comparing the rAAV5 product containing the capsid protein VP1 comprising the mutations T614A, S2A and T711S (AAV5-03Mut-GFP) and capsid VP1 comprising only the mutations S2A and T711S (AAV5-01Mut-GFP), there was no statistically significant changes in the number of packaged viral genomes.

In the presence of the mutations G226V, S2A and T711S (AAV5-04Mut-GFP), the number of packaged viral genomes increased by 6.77 times from 2.51E+09 vg/ml to 1.70E+10 vg/ml compared to the control rAAV5 product with the wild-type capsid protein VP1 (AAV5-NullMut-GFP). When comparing the rAAV5 product containing the capsid protein VP1 comprising the mutations G226V, S2A and T711S (AAV5-04Mut-GFP) and capsid VP1 comprising only the mutations S2A and T711S (AAV5-01Mut-GFP), the number of packaged viral genomes increased 1.25 times from 1.36E+10 vg/ml to 1.70E+10 vg/ml.

## Claims

1. An isolated modified capsid protein VP1 from adeno-associated virus serotype 5 (AAV5) for production of viral vectors based on recombinant adeno-associated virus serotype 5 (rAAV5), said isolated modified capsid protein VP1 comprising a wild-type AAV5 capsid protein VP1 amino acid sequence encoded by the *Cap* gene with one or more substitutions that are selected from the group:
G226V,
S2A, G226V and T711S,
T614A,
S2A, T614A and T711S,
T614V, or
S2A, T614V and T711S,
wherein the wild-type AAV5 capsid protein VP1 amino acid sequence has the amino acid sequence represented by SEQ ID NO: 1.

2. The isolated modified AAV5 capsid protein VP1 according to claim 1, comprising a substitution at position G226V.

3. The isolated modified AAV5 capsid protein VP1 according to claim 2, having the amino acid sequence represented by SEQ ID NO: 3.

4. The isolated modified AAV5 capsid protein VP1 according to claim 1, comprising the substitutions S2A, G226V and T711S.

5. The isolated modified AAV5 capsid protein VP1 according to claim 4, having the amino acid sequence represented by SEQ ID NO: 4.

6. The isolated modified AAV5 capsid protein VP1 according to claim 1, comprising the substitution T614A.

7. The isolated modified AAV5 capsid protein VP1 according to claim 6, having the amino acid sequence represented by SEQ ID NO: 5.

8. The isolated modified AAV5 capsid protein VP1 according to claim 1, comprising the substitutions S2A, T614A and T711S.

9. The isolated modified AAV5 capsid protein VP1 according to claim 8, having the amino acid sequence represented by SEQ ID NO: 6.

10. The isolated modified AAV5 capsid protein VP1 according to claim 1, comprising the substitution T614V.

11. The isolated modified AAV5 capsid protein VP1 according to claim 10, having the amino acid sequence represented by SEQ ID NO: 7.

12. The isolated modified AAV5 capsid protein VP1 according to claim 1, comprising the substitutions S2A, T614V and T71 1S.

13. The isolated modified AAV5 capsid protein VP1 according to claim 12, having the amino acid sequence represented by SEQ ID NO: 8.

14. An isolated nucleic acid encoding the modified capsid protein VP1 from adeno-associated virus serotype 5 according to any one of claims 1 to 13, said capsid protein VP1 being used for production of viral vectors based on recombinant adeno-associated virus serotype 5.

15. The isolated nucleic acid according to claim 14 encoding a modified capsid protein VP1 from adeno-associated virus serotype 5 with the amino acid substitution G226V, said isolated nucleic acid being represented by a nucleic sequence with SEQ ID NO: 11 or by any other sequence encoding the respective amino acid sequence.

16. The isolated nucleic acid according to claim 14 encoding a modified capsid protein VP1 from adeno-associated virus serotype 5 with the amino acid substitutions S2A, G226V and T711S, said isolated nucleic acid being represented by a nucleic sequence with SEQ ID NO: 12 or by any other sequence encoding the respective amino acid sequence.

17. The isolated nucleic acid according to claim 14 encoding a modified capsid protein VP1 from adeno-associated virus serotype 5 with the amino acid substitution T614A, said isolated nucleic acid being represented by a nucleic sequence with SEQ ID NO: 13 or by any other sequence encoding the respective amino acid sequence.

18. The isolated nucleic acid according to claim 14 encoding a modified capsid protein VP1 from adeno-associated virus serotype 5 with the amino acid substitutions S2A, T614A and T711S, said isolated nucleic acid being represented by a nucleic sequence with SEQ ID NO: 14 or by any other sequence encoding the respective amino acid sequence.

19. The isolated nucleic acid according to claim 14 encoding a modified capsid protein VP1 from adeno-associated virus serotype 5 with the amino acid substitution T614V, said isolated nucleic acid being represented by a nucleic sequence with SEQ ID NO: 15 or by any other sequence encoding the respective amino acid sequence.

20. The isolated nucleic acid according to claim 14 encoding a modified capsid protein VP1 from adeno-associated virus serotype 5 with the amino acid substitutions S2A, T614V and T711S, said isolated nucleic acid being represented by a nucleic sequence with SEQ ID NO: 16 or by any other sequence encoding the respective amino acid sequence.

21. An isolated capsid for production of viral vectors based on recombinant adeno-associated virus serotype 5, said isolated capsid including the modified capsid protein VP1 from adeno-associated virus serotype 5 according to any one of claims 1 to 13.

22. The isolated capsid according to claim 21 comprising the modified capsid protein VP1 from adeno-associated virus serotype 5 according to any one of claims 1 to 13, the AAV5 capsid protein VP2 or a modified variant thereof, and the AAV5 capsid protein VP3 or a modified variant thereof.

23. The isolated capsid according to claim 22 comprising the wild-type AAV5 capsid protein VP2.

24. The isolated capsid according to claim 23 comprising the wild-type AAV5 capsid protein VP2 that has an amino acid sequence represented by SEQ ID NO: 17.

25. The isolated capsid according to claim 22 comprising a modified capsid protein VP2 from adeno-associated virus serotype 5.

26. The isolated capsid according to claim 25 comprising a modified AAV5 capsid protein VP2 comprising the substitution G90V.

27. The isolated capsid according to claim 26 comprising a modified AAV5 capsid protein VP2 comprising the substitution G90V and having an amino acid sequence represented by SEQ ID NO: 19.

28. The isolated capsid according to claim 25 comprising a modified AAV5 capsid protein VP2 comprising the substitutions G90V and T575S.

29. The isolated capsid according to claim 28 comprising a modified AAV5 capsid protein VP2 comprising the substitutions G90V and T575S and having an amino acid sequence represented by SEQ ID NO: 20.

30. The isolated capsid according to claim 25 comprising a modified AAV5 capsid protein VP2 comprising the substitution T478A.

31. The isolated capsid according to claim 30 comprising a modified AAV5 capsid protein VP2 comprising the substitution T478A and having an amino acid sequence represented by SEQ ID NO: 21.

32. The isolated capsid according to claim 25 comprising a modified AAV5 capsid protein VP2 comprising the substitutions T478A and T575S.

33. The isolated capsid according to claim 32 comprising a modified AAV5 capsid protein VP2 comprising the substitutions T478A and T575S and having an amino acid sequence represented by SEQ ID NO: 22.

34. The isolated capsid according to claim 25 comprising a modified AAV5 capsid protein VP2 comprising the substitution T478V.

35. The isolated capsid according to claim 34 comprising a modified AAV5 capsid protein VP2 comprising the substitution T478V and having an amino acid sequence represented by SEQ ID NO: 23.

36. The isolated capsid according to claim 25 comprising a modified AAV5 capsid protein VP2 comprising the substitutions T478V and T575S.

37. The isolated capsid according to claim 36 comprising a modified AAV5 capsid protein VP2 comprising the substitutions T478V and T575S and having an amino acid sequence represented by SEQ ID NO: 24.

38. The isolated capsid according to claim 22 comprising the wild-type AAV5 capsid protein VP3.

39. The isolated capsid according to claim 38 comprising the wild-type AAV5 capsid protein VP3 that has an amino acid sequence represented by SEQ ID NO: 33.

40. The isolated capsid according to claim 22 comprising a modified capsid protein VP3 from adeno-associated virus serotype 5.

41. The isolated capsid according to claim 40 comprising a modified AAV5 capsid protein VP3 comprising the substitution G34V.

42. The isolated capsid according to claim 41 comprising a modified AAV5 capsid protein VP3 comprising the substitution G34V and having an amino acid sequence represented by SEQ ID NO: 35.

43. The isolated capsid according to claim 40 comprising a modified AAV5 capsid protein VP3 comprising the substitutions G34V and T519S.

44. The isolated capsid according to claim 43 comprising a modified AAV5 capsid protein VP3 comprising the substitutions G34V and T519S and having an amino acid sequence represented by SEQ ID NO: 36.

45. The isolated capsid according to claim 40 comprising a modified AAV5 capsid protein VP3 comprising the substitution T422A.

46. The isolated capsid according to claim 45 comprising a modified AAV5 capsid protein VP3 comprising the substitution T422A and having an amino acid sequence represented by SEQ ID NO: 37.

47. The isolated capsid according to claim 40 comprising a modified AAV5 capsid protein VP3 comprising the substitutions T422A and T519S.

48. The isolated capsid according to claim 47 comprising a modified AAV5 capsid protein VP3 comprising the substitutions T422A and T519S and having an amino acid sequence represented by SEQ ID NO: 38.

49. The isolated capsid according to claim 40 comprising a modified AAV5 capsid protein VP3 comprising the substitution T422V.

50. The isolated capsid according to claim 49 comprising a modified AAV5 capsid protein VP3 comprising the substitution T422V and having an amino acid sequence represented by SEQ ID NO: 39.

51. The isolated capsid according to claim 40 comprising a modified AAV5 capsid protein VP3 comprising the substitutions T422V and T519S.

52. The isolated capsid according to claim 51 comprising a modified AAV5 capsid protein VP3 comprising the substitutions T422V and T519S and having an amino acid sequence represented by SEQ ID NO: 40.

53. An isolated nucleic acid encoding the capsid according to any one of claims 21 to 52, said capsid being used for production of viral vectors based on recombinant adeno-associated virus serotype 5.

54. A vector based on recombinant adeno-associated virus serotype 5 for delivering a heterologous nucleic acid sequence to a subject, said vector comprising:
1) the modified capsid protein VP1 from adeno-associated virus serotype 5 according to any one of claims 1 to 13 or the capsid according to any one of claims 21 to 52, and
2) a heterologous nucleic acid sequence comprising regulatory sequences that promote the expression of the product encoded by the heterologous nucleic acid sequence, in the target cells.

55. The rAAV5 vector according to claim 54, wherein the product of expression of the heterologous nucleic acid sequence is a therapeutic polypeptide or reporter polypeptide.

56. A pharmaceutical composition for the delivery of a gene product to a subject in need thereof, comprising:
a) the rAAV5 vector according to any one of claims 54 to 55; and
b) a pharmaceutically acceptable excipient.

57. A method for delivering a gene product to a subject in need thereof, comprising administering to the subject the rAAV5 vector according to any one of claims 54 to 55 or the pharmaceutical composition according to claim 56.

58. Use of the rAAV5 vector according to any one of claims 54 to 55 or the pharmaceutical composition according to claim 56 for treating a disease in a subject in need thereof.

59. The use according to claim 58, wherein the disease is selected from the group: blood diseases; central nervous system diseases; metabolic diseases; muscle diseases; hereditary diseases.

60. A method for producing the rAAV5 vector according to any one of claims 54 to 55, comprising transfection of producer cells with the nucleic acid encoding a modified capsid protein VP1 from adeno-associated virus serotype 5 according to any one of claims 14 to 20, or with the nucleic acid encoding a capsid according to claim 53.
